(19) Europäisches Patentamt
European Patent Office
Office européen des brevets

(11) Publication number: 0 220 194 B1

# EUROPEAN PATENT SPECIFICATION

(45) Date of publication of patent specification: 30.03.94  (51) Int. Cl.5: **A61N 1/365**

(21) Application number: **85905293.8**

(22) Date of filing: **11.10.85**

(86) International application number:
**PCT/US85/02010**

(87) International publication number:
**WO 86/05698 (09.10.86 86/22)**

(54) **STIMULATED HEART INTERVAL MEASUREMENT, ADAPTIVE PACER AND METHOD OF OPERATION.**

(30) Priority: **27.03.85 US 716831**

(43) Date of publication of application:
**06.05.87 Bulletin 87/19**

(45) Publication of the grant of the patent:
**30.03.94 Bulletin 94/13**

(84) Designated Contracting States:
**DE FR GB IT NL SE**

(56) References cited:
EP-A- 0 007 189     EP-A- 0 050 038
EP-A- 0 063 097     DE-A- 2 252 312
US-A- 3 857 399     US-A- 4 060 090
US-A- 4 305 396     US-A- 4 312 355
US-A- 4 313 442     US-A- 4 373 531
US-A- 4 401 119     US-A- 4 527 568

IEEE Transactions on Biomedical Engineering, vol.BME-29 (1982), no.5, May 1982, pages 359-361, New York US, A.R.S.Bukhari et al: "Cardiac arrival time analyzer"

(73) Proprietor: **Siemens Elema AB**

Röntgenvägen 2
S-171 95 Solna 1(SE)

(84) Designated Contracting States:
**SE**

(73) Proprietor: **SIEMENS AKTIENGESELLSCHAFT**
**Wittelsbacherplatz 2**
**D-80333 München(DE)**

(84) Designated Contracting States:
**DE FR GB IT NL**

(72) Inventor: **THORNANDER, Hans, T.**
**20 rue des Ecules**
**F-75005 Paris(FR)**
Inventor: **POORE, John, W.**
**9442 Farraline Ave**
**Chatsworth, CA 91311(US)**
Inventor: **SHOLDER, Jason, A.**
**21037 Cantara Street**
**Canoga Park, CA 91304(US)**
Inventor: **THACKER, James, R.**
**28613 Crown Court**
**Saugus, CA 91350(US)**

**Description**

Background

The heart is a pump that pumps blood throughout the body. Cardiac pacemakers have long been used to provide stimulation pulses to the heart in order to control the rate at which the heart pumps or beats, thereby controlling the flow rate at which blood is circulated throughout the body. The principal purpose for circulating blood throughout the body, of course, is to deliver oxygen and other nutrients to the body cells, without which oxygen the body cells would soon die. As the body cells are called upon to do more and more work, the flow rate at which oxygenated blood is delivered to the cells must be increased. This increase in flow rate can be achieved by increasing the rate at which the heart beats or pumps. In a normal, healthy, nonpaced heart, the heart rate automatically increases in response to the need to deliver more oxygenated blood to the body cells. However, a pacemaker-controlled heart is unable to automatically increase its rate unless the pacemaker is able to sense that an increased oxygen need is present.

Modern pacemakers include complex stimulation pulse generators as well as cardiac event sensors that can pace or sense in the atrium, the ventricle, or both the atrium and ventricle of the heart. Further, such pacemakers include telemetry capabilities so that the activity of the heart and pacemaker can be transmitted to an attending physician or cardiologist. Advantageously, such pacemakers are also programmable so that the same telemetry capabilities can be used by the attending physician or cardiologist in order to adjust the control parameters associated with operation of the pacemaker. Such parameters not only influence the rate at which the pacemaker's stimulation pulses are generated, but also control the pacemaker's basic mode of operation, i.e., the heart chamber that is paced, as well as the heart chamber that is sensed. Hence, modern pacemakers offer great versitility in the manner of their use. Disadvantageously, many modern pacemakers do not yet have the capability to automatically adjust the pacing rate, or pacing interval, in the absence of a sinus P-wave (a sinus P-wave is explained below) as a function of the body's physiological needs unless some sort of physiological sensor external to the pacemaker is employed. As used herein, the term "physiological need" includes the need to change the flow rate at which oxygenated blood is delivered to the body's cells, as well as other body needs that influence the heart rate.

The present invention is directed to an improved pacemaker that includes the capability of automatically adjusting the paced heart rate as a function of sensed physiologiocal needs within the body. Advantageously, no electronic sensors external to the pacemaker need be employed beyond the normal stimulation leads that are connected between the pacemaker and the heart. As is explained more fully below, the present invention senses physiological need by noting changes in a selected time interval associated with the rhythm of the heart.

EP-A-0007189 discloses a rate adaptive pacemaker employing the measurement of a selected time interval associated with the heart rythm for sensing a patient's workload. The time interval selected for rate control in this prior art pacemaker is generally referred to as the Q-T interval, and the interval specifically measured is that between a ventricular stimulation pulse and the corresponding repolarization ventricular cardiac event (T-wave).

In contrast, and as fully explained below, the time interval relied upon according to the present invention for rate control is the physiologically different interval between a cardiac stimulation pulse and a depolarization cardiac event.

SUMMARY

The invention is defined in the claims.

While the scope of the invention thus should be determined with respect to the claims, the invention is now explained in general terms as an introduction to the thereupon following detailed description of the best contemplated mode for carrying out the invention.

The present invention provides a physiological sensor that can sense when the heart rate needs to increase or decrease as a function of the physiological need of the body within which the heart is located, and a means for automatically adjusting the pacing rate controlled by a pacemaker as a function of said physiological need; i.e. a means for automatically adjusting the escape interval associated with a demand-type pacemaker, which escape interval defines the time interval within which a natural heart event (such as an atrial or ventricular depolarization) must occur in order to inhibit the delivery of a stimulating pulse to the heart, said escape interval being adjusted by the adjustment means of the present invention so as to increase or decrease the rate at which the heart beats in accordance with said physiological need, which need is established by means for selectively measuring the time interval between a stimulating pulse applied to the atrium or ventricle of the heart and a responsive cardiac event, such as an atrial or ventricular depolarization or contraction, and means for using this measured time interval as a control parameter that adjusts the pacing rate of the pacemaker.

Preferably, the pacemaker further includes means for processing the previously measured time intervals (from the past several heart cycles) in order to generate a reference control parameter that smoothly and safely effectuates a pacing rate change.

The physiological sensor of the present invention is premised on the discovery that the time interval between application of a stimulating pulse to the atrium or ventricle of a heart and a resulting cardiac event, which event could be either the resulting atrial depolarization or the ventricular depolarization (which ventricular depolarization could, in turn, be related to the depolarization that follows an atrial depolarization or that results from a ventricular stimulating pulse) varies as a function of the physiological need of the body within which the heart is located. In one embodiment to be described, therefore, the sensor of the present invention measures the time interval between application of an atrial stimulation pulse, or "A-pulse", and the occurrence of a responsive atrial depolarization, or "P-wave" (the occurrence of a P-wave indicating depolarization of the atrium). In this embodiment, the time interval measured is designated as the A-P interval, or API. In a second embodiment to be described, available for use when AV conduction of the heart is not blocked, the sensor of the present invention measures the time interval between application of the A-pulse and the subsequent occurrence of a ventricular depolarization or "R-wave" (the occurrence of an R-wave indicating depolarization of the ventricle). In the second embodiment, the time interval measured is designated as the A-R interval or ARI. In a third embodiment to be described, primarily for use with a single chamber pacer that senses and pulses in the ventricle (or a dual chambered pacer programmed to operate only in a single chamber mode), the sensor measures the time interval between application of a ventricular stimulation pulse, or "V-pulse," and the occurrence of the responsive ventricular depolarization, or R-wave. In this embodiment, the time interval measured is designated as the V-R interval, or VRI.

The physiological sensor of the present invention therefore includes time interval measurement means for measuring the A-P, A-R, V-R intervals. Preferably these interval measurements are smoothed, averaged, or otherwise processed through appropriate processing means to produce a reference interval measurement that is derived from the combined interval measurements of the past several heart cycles. As such, this reference interval measurement is free of abrupt changes, and any established trend in the lengthening or shortening thereof can be safely interpreted as a change in the physiological need of the body.

In a demand-type pacemaker, wherein a stimulating pulse is provided by the pacemaker only when a natural cardiac event fails to occur within a prescribed escape time interval, the present invention adjusts the escape time interval as a function of the reference interval measurement, and thereby effectuates the same desired result of an adjustable pacing rate as a funtion of physiological need. These escape time intervals are typically subsets of the longer A-V interval, and V-A interval, the sum of which defines the pacing interval controlled by the pacemaker. Accordingly, the reference interval measurement can be used to adjust the pacemaker-controlled A-V interval, the V-A interval, or both the A-V interval and the V-A interval, thereby controlling the pacing rate. The reference interval measurement generated by the present invention may also be used to control other parameters associated with the operation of the pacemaker in order to render the pacemaker more physiologically responsive.

In accordance with one described embodiment of the physiological sensor of the present invention, used in conjunction with an demand-type pacemaker, the time interval measurements, e.g. A-P or A-R, are processed as follows:

At least three (3) previous heart cycles are monitored. If an A-pulse was inhibited more than once during the previous consecutive three (3) cycles, then the pacing rate does not change; if, however, at least 3 A-pulses have been generated and at least three API or ARI measurements have successfully been made, then the API or ARI measurements over these previous three cycles are examined to determine if all are less than or greater than a reference interval measurement, which reference interval measurement is representative of the current pacing rate. If an increasing or decreasing trend is noted, that is, if all three previous consecutive interval measurements are moving in the same direction, then the interval measurement closest to the reference interval measurement is used as the new reference interval measurement.

In another described embodiment of the physiological sensor, the time interval measurements may be smoothed through averaging an appropriate number of measurements from prior cardiac cycles.

An important requisite for measuring the A-P interval is the ability to sense a paced P-wave or stimulated atrial repolarization. While various techniques may be used to sense such a P-wave, the preferred mode for carrying out the present invention contemplates a unipolar use of a conventional atrial-placed bipolar lead. In accordance with this technique, atrial stimulation occurs through unipolar exitation of the atrium through the distal tip of the conventional bipolar lead. P-wave sensing occurs through unipolar sensing, ring-to-case, of the P-wave generated by the atrium as depolarization occurs. Utilizing the spaced-apart distal tip and ring of the conventional bipolar lead in a unipolar mode of operation allows the P-event, occuring within a relatively short time after the generation of the A-pulse, to be accurately sensed. Alternatively, separate unipolar leads may be selectively placed within the atrium, spaced one apart from the other, in order to serve the same function. Further, for a single chamber pacer connected only to the ventricle, these same techniques may be used to sense the R-wave that occurs in response to an applied V-pulse.

## BRIEF DESCRIPTION OF THE DRAWINGS

The above and other objects, features and advantages of the present invention will be more apparent from the following more particular description thereof presented in conjunction with the following drawings, wherein:

Fig. 1 is a schematic representation of a human heart illustrating the main components thereof and the flow of blood therethough;

Fig. 2 is a simplified representation of the heart showing the location of the SA and AV nodes;

Fig. 3 is a timing diagram illustrating the normal, non-paced operation of the heart of Fig. 2 as sensed through conventional skin ECG electrodes or equivalent;

Fig. 4 is a simplified representation of the heart showing the manner in which a pacemaker is connected thereto through insertion of bipolar leads into both the right atrium and right ventricle;

Fig. 5 is a timing diagram showing the relationship between pacing pulses delivered to the heart from a pacemaker and the heart's response to these pacing pulses;

Fig. 6 is a timing diagram similar to Fig. 5 showing a P-wave that occurs in response to an atrium stimulation pulse, followed by a natural (non-paced) ventricular R-event, and further showing consecutive A-P and A-R time intervals that occur over consecutive pacing intervals;

Figs. 7A and 7B are timing diagrams as in Figs. 5 and 6, but showing some different possible sequences of cardiac events and further defining various time intervals that are used in the operation of a dual-chamber demand-type pacemaker;

Fig. 8 is a timing diagram with an expanded time base that illustrates variations of the A-P/A-R interval time measurements that may be utilized as part of the invention;

Fig. 9A is a simplified representation of the heart showing how a plurality of unipolar leads could be positioned within the atrium chamber of the heart for use with the present invention as an alternative to the atrial bipolar lead shown in Fig. 4;

Fig. 9B is a timing diagram illustrating the sequence of P-waves that are sensed using the unipolar leads of Fig. 9A;

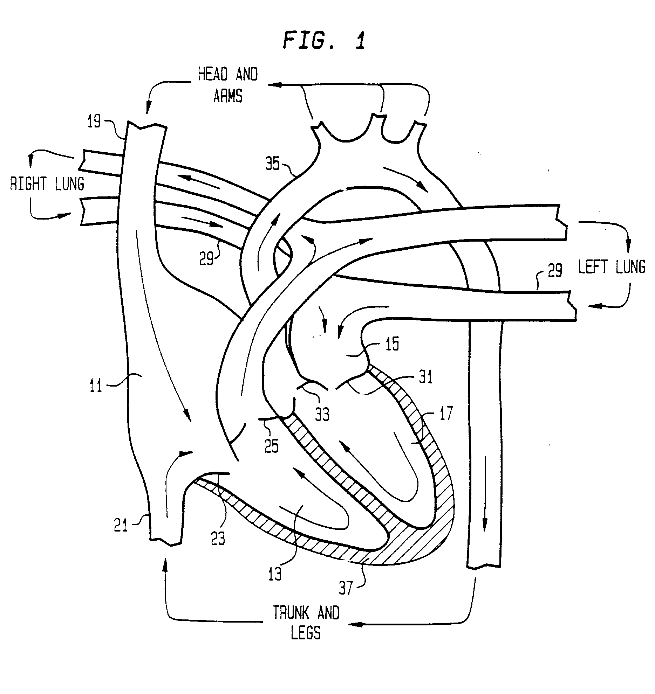

Fig. 1∅ is a block diagram of the pacemaker system of the present invention;

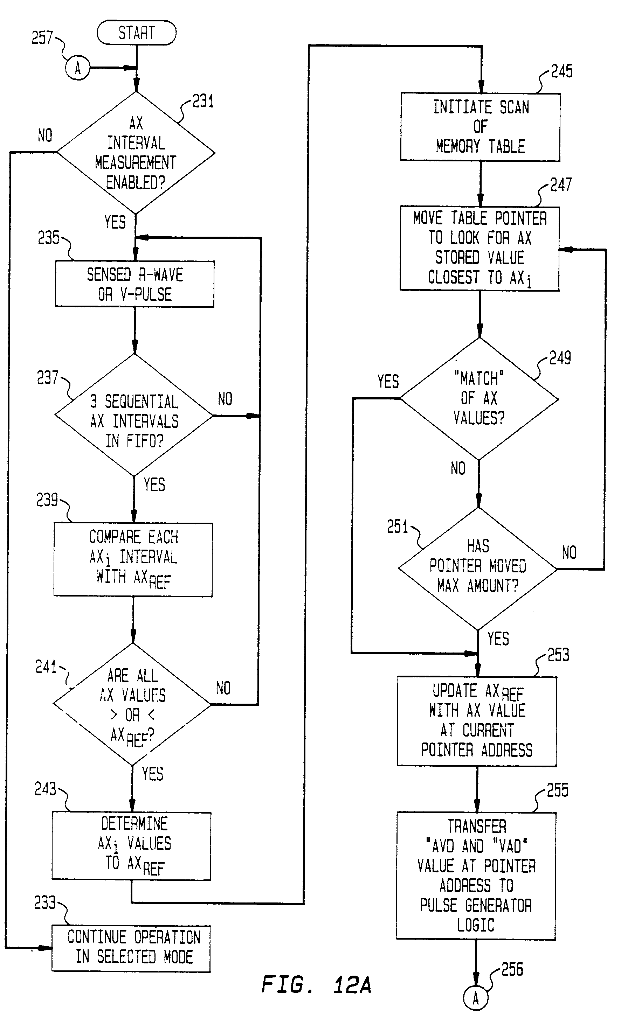

Fig. 11 is a block diagram of the physiological detector of Fig. 1∅;

Fig. 12A is a flow diagram illustrating the process used by the control logic of Fig. 11 in converting the time interval measurement to control parameters for delivery to the pulse generator logic;

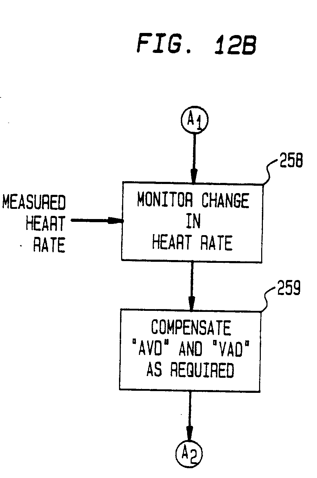

Fig. 12B is an extension of Fig. 12A for one embodiment of the invention;

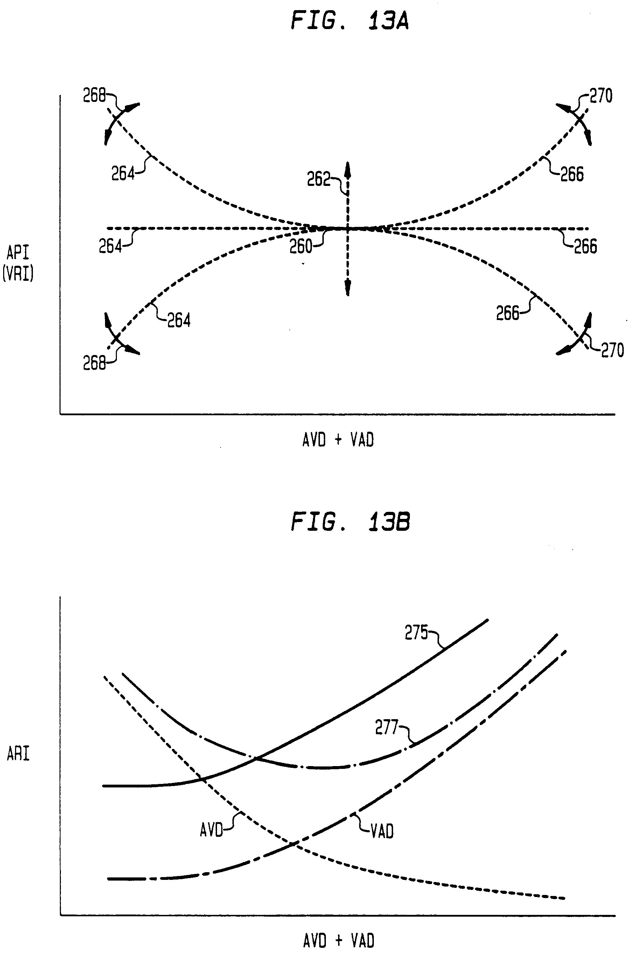

Figs. 13A and 13B are graphs depicting illustrative relationships between the A-P and A-R intervals and the AVD and VAD (A-V delay and V-A delay) control parameters that could be established by the present invention;

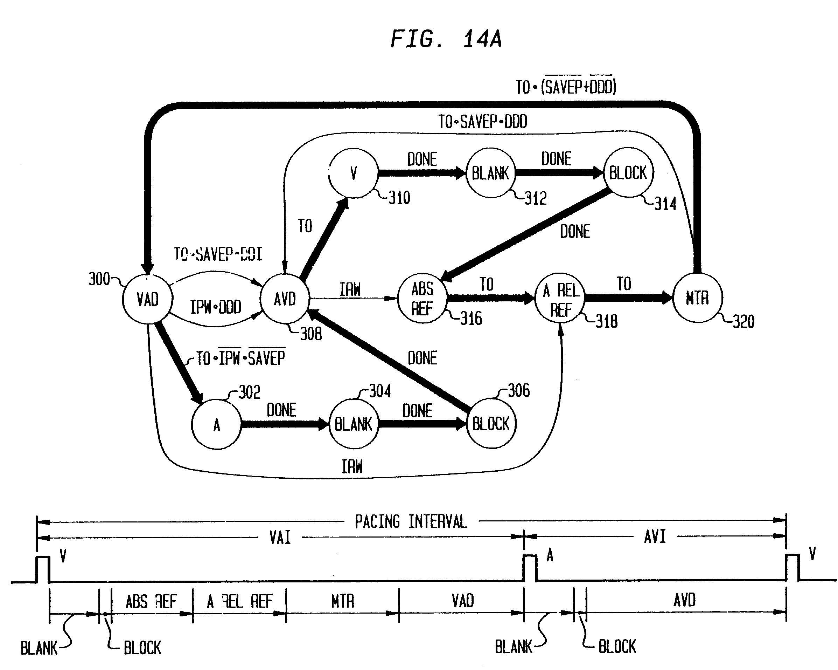

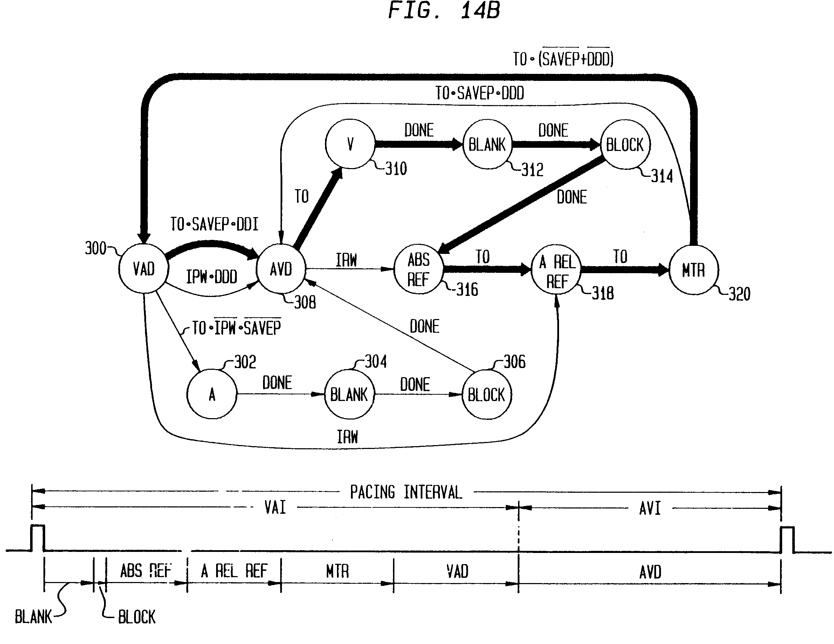

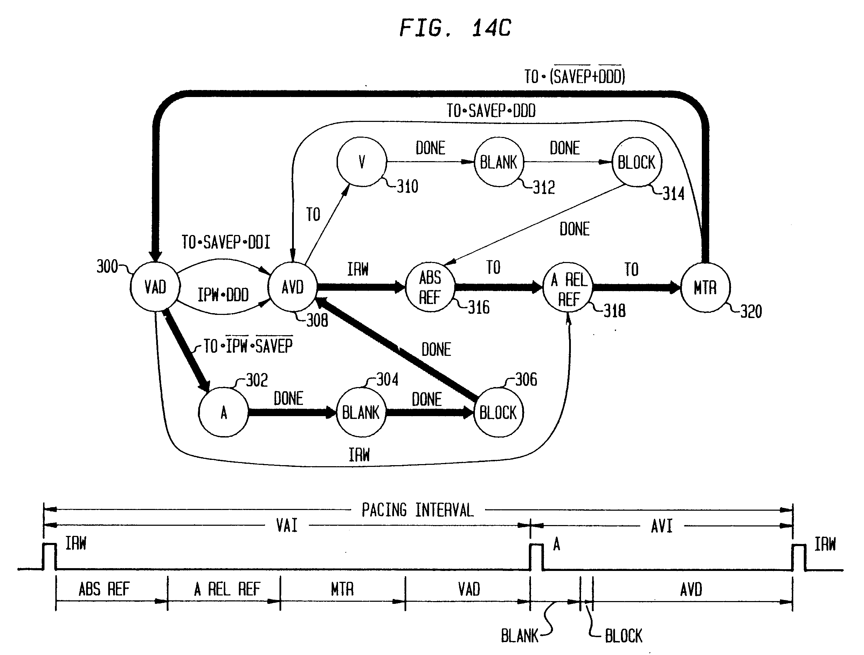

Figs. 14A - 14C are state diagrams illustrating possible operating states associated with the pulse generator logic of Fig. 1∅;

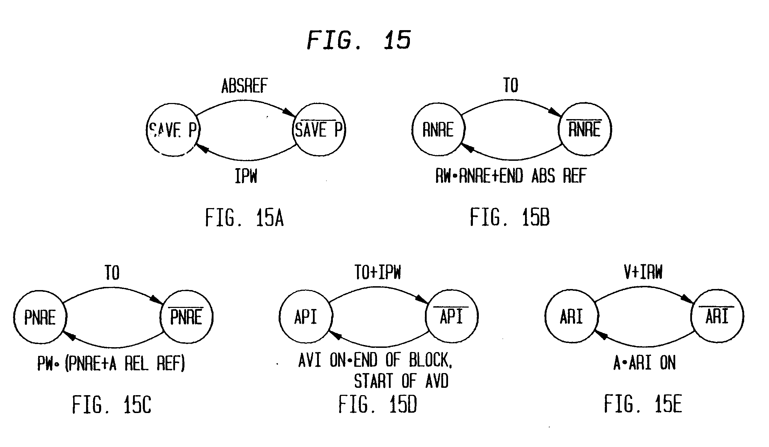

Figs. 15A - 15E are further state diagrams as in Figs. 14A - 14C.

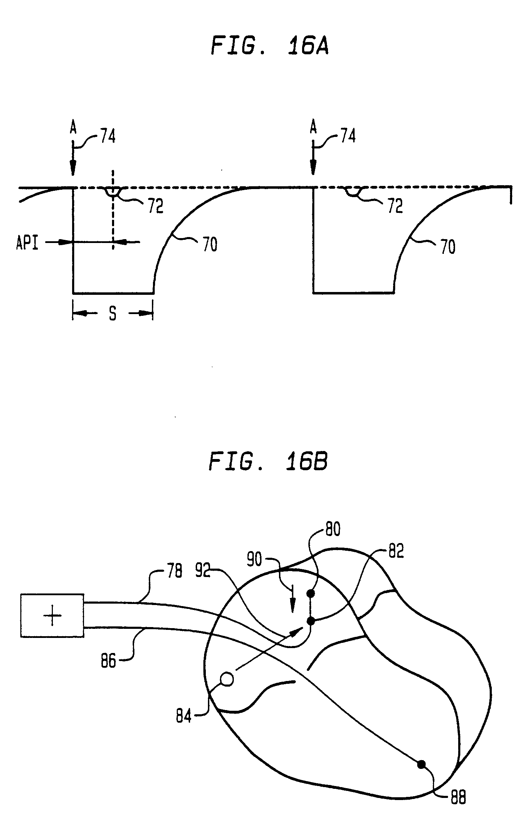

Fig. 16A is a waveform diagram illustrating the problem of P-wave detection utilizing the atrial stimulation electrode as the P-wave sensing electrode;

Fig 16B is a schematic representation of a user's heart showing locations of the atrial and ventricle electrodes;

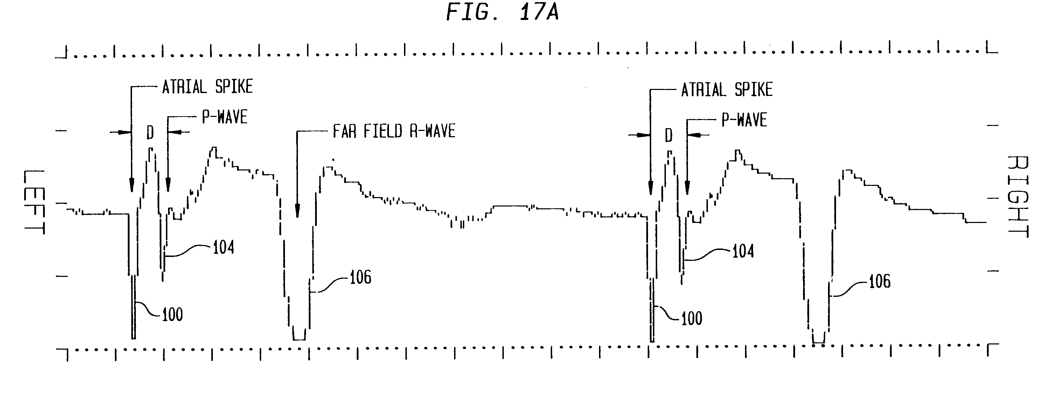

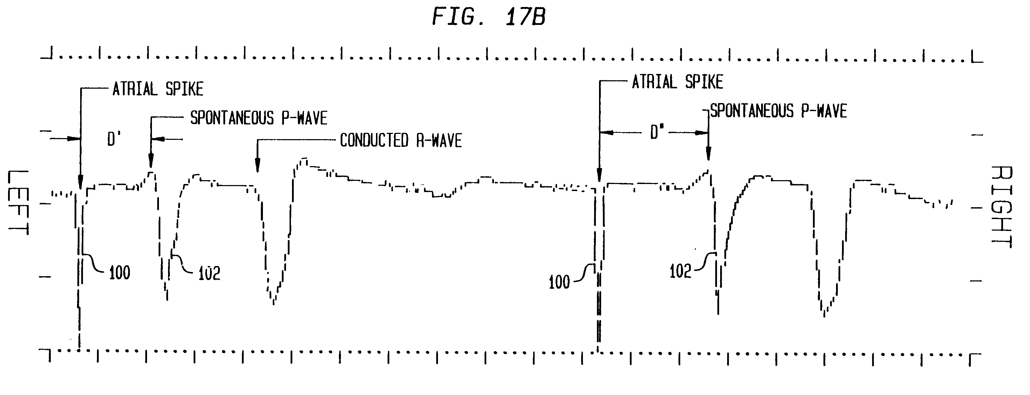

Figs. 17A and 17B are waveform diagrams of intercardiac electrogram (EGM) signals, Fig. 17A illustrating an EGM signal showing P-wave capture and Fig. 17B illustrating an EGM signal in the absence of P-wave capture;

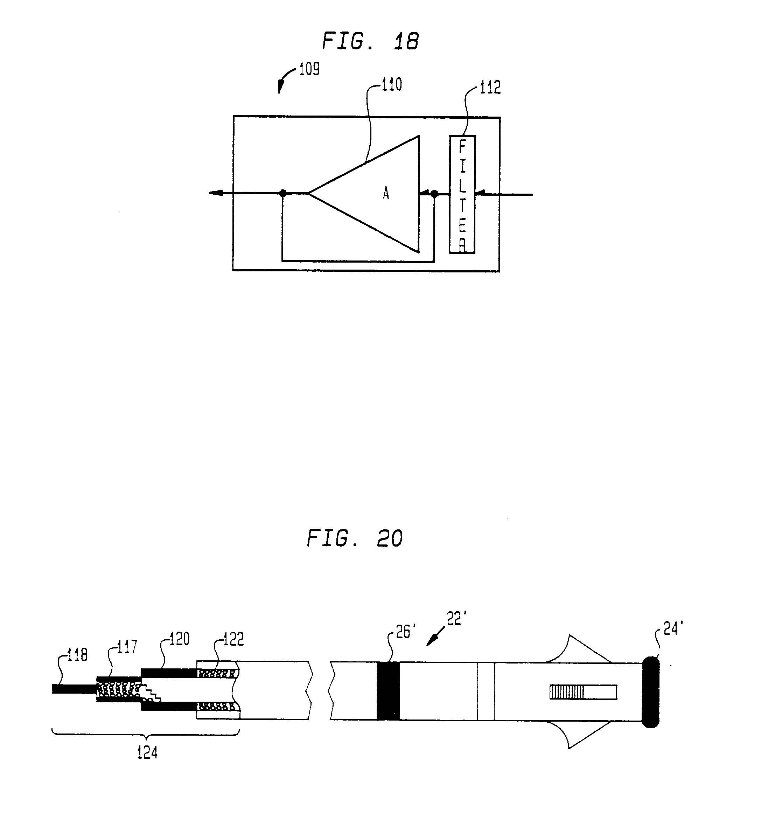

Fig. 18 is a block diagram of a P-wave amplifier as shown in Fig. 1∅;

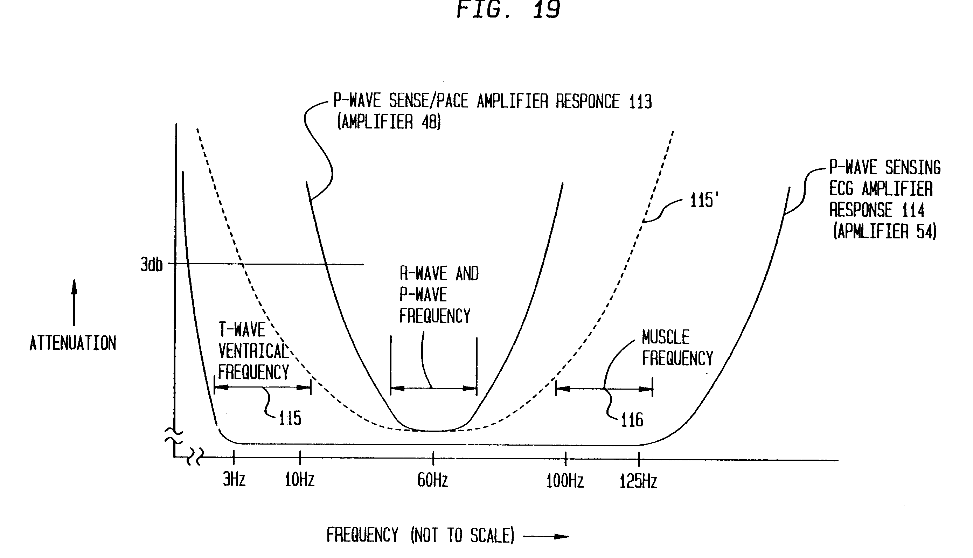

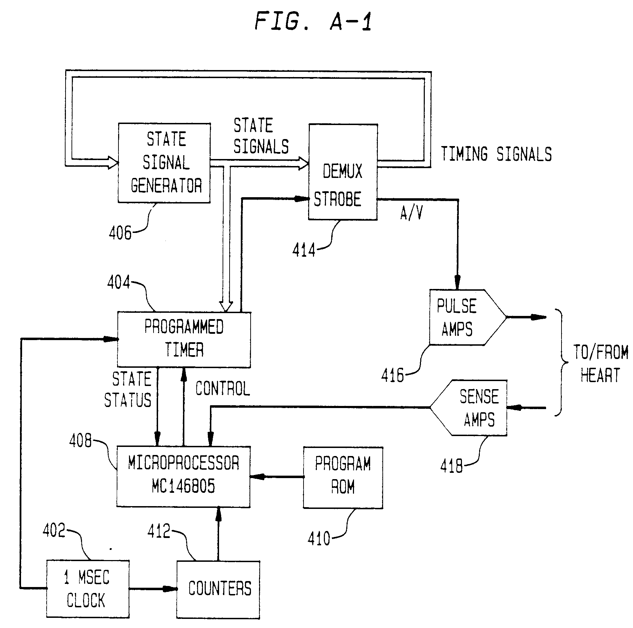

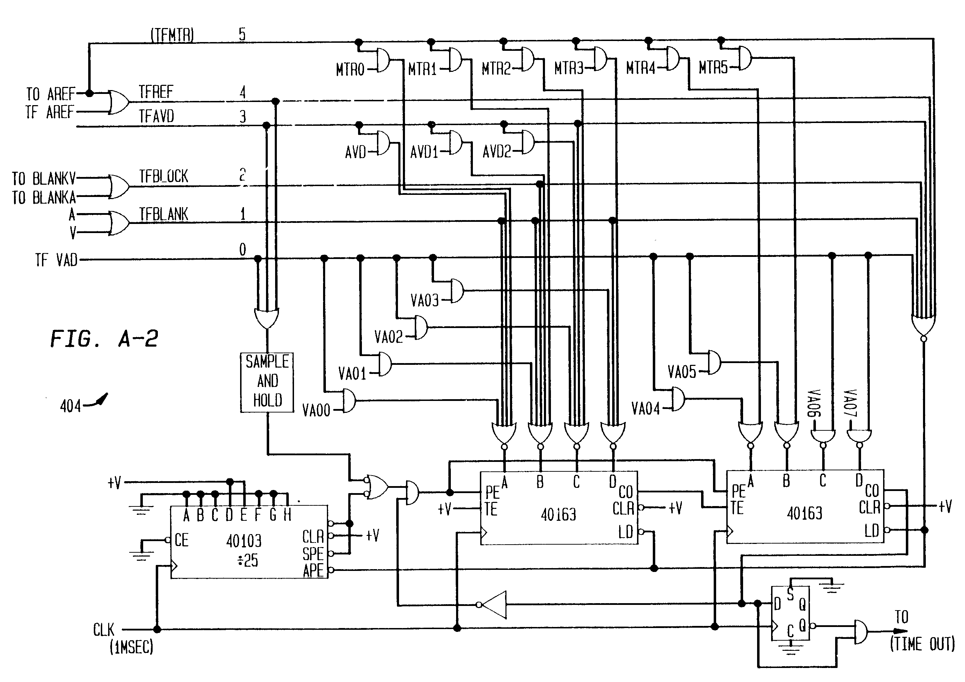

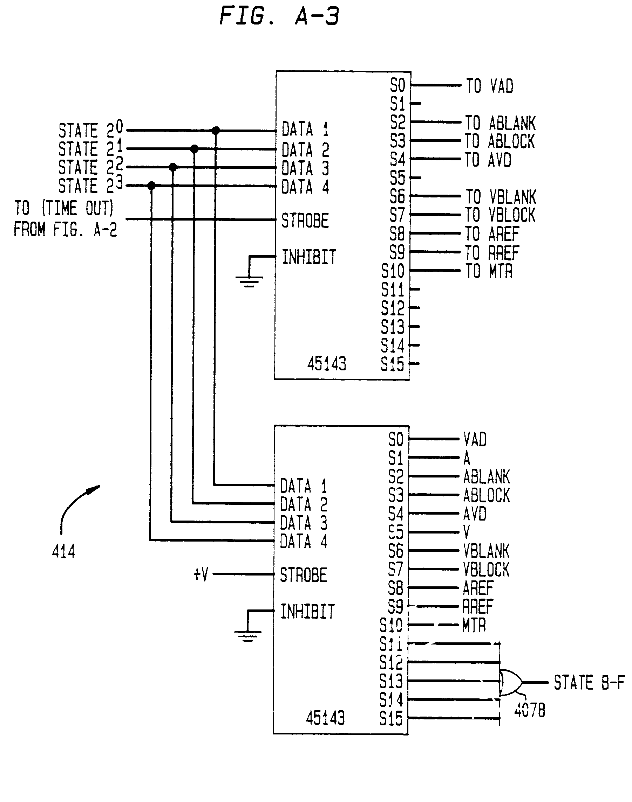

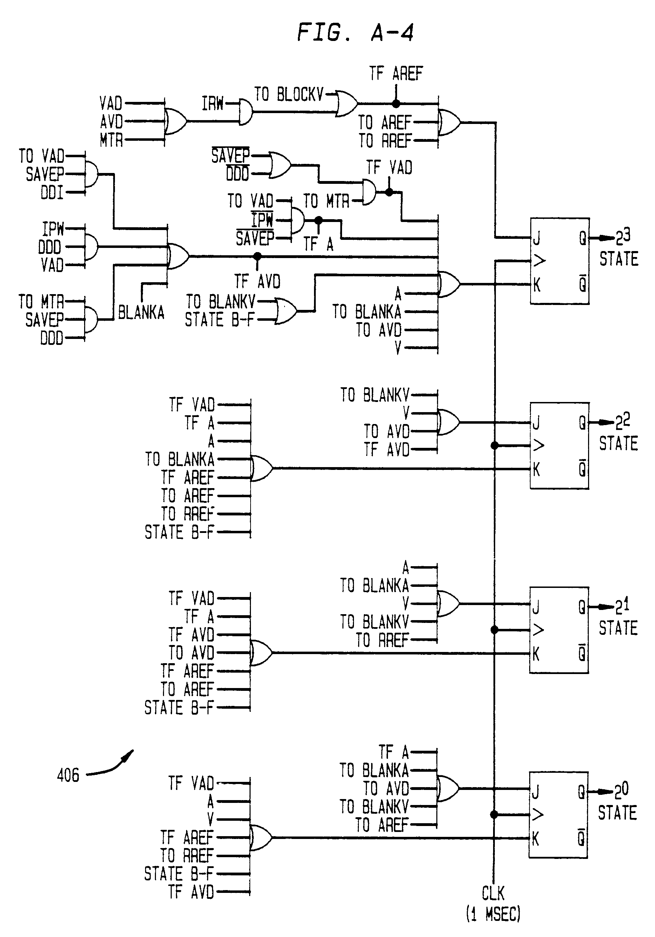

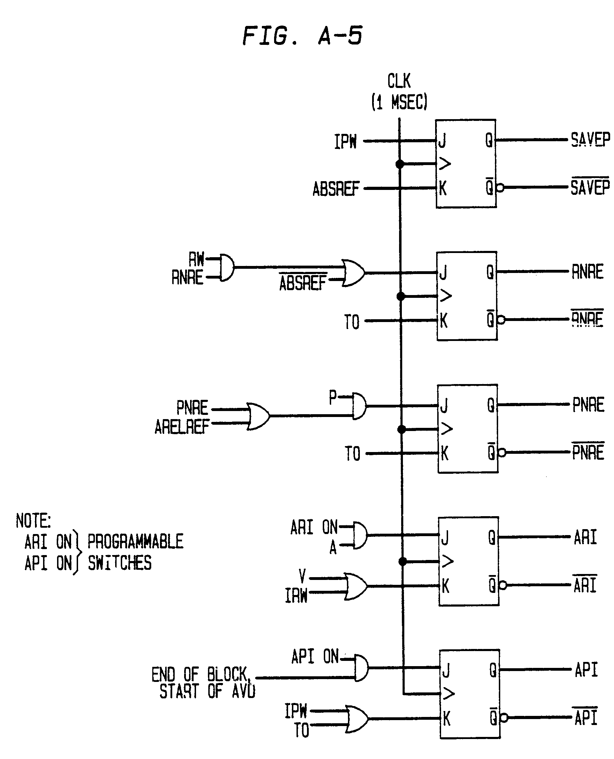

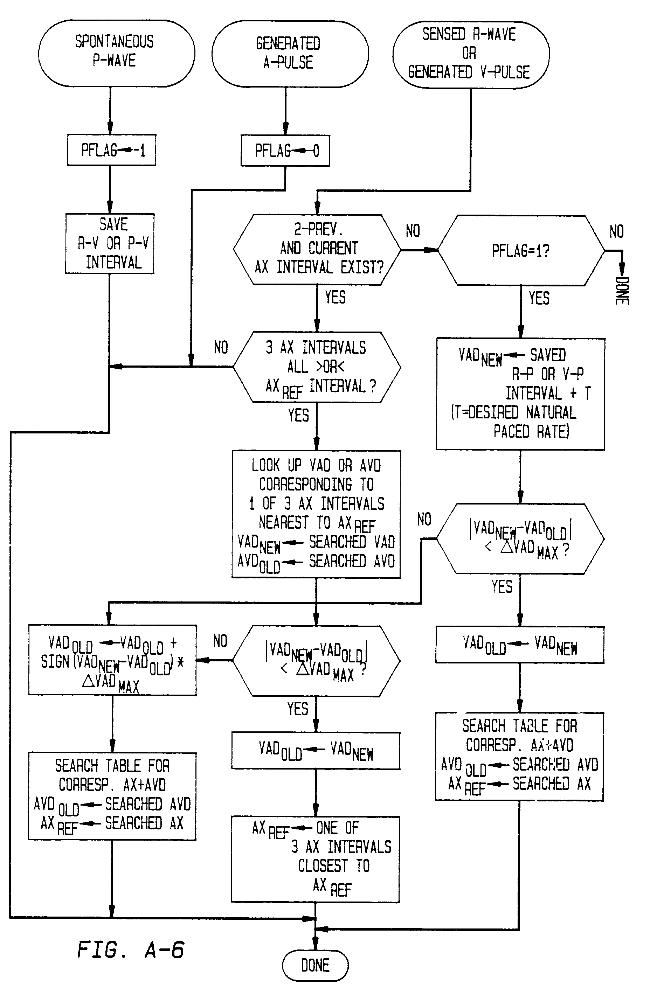

Fig. 19 shows frequency response curves for the P-wave sense/amplifier and the P-wave sensing amplifier; and

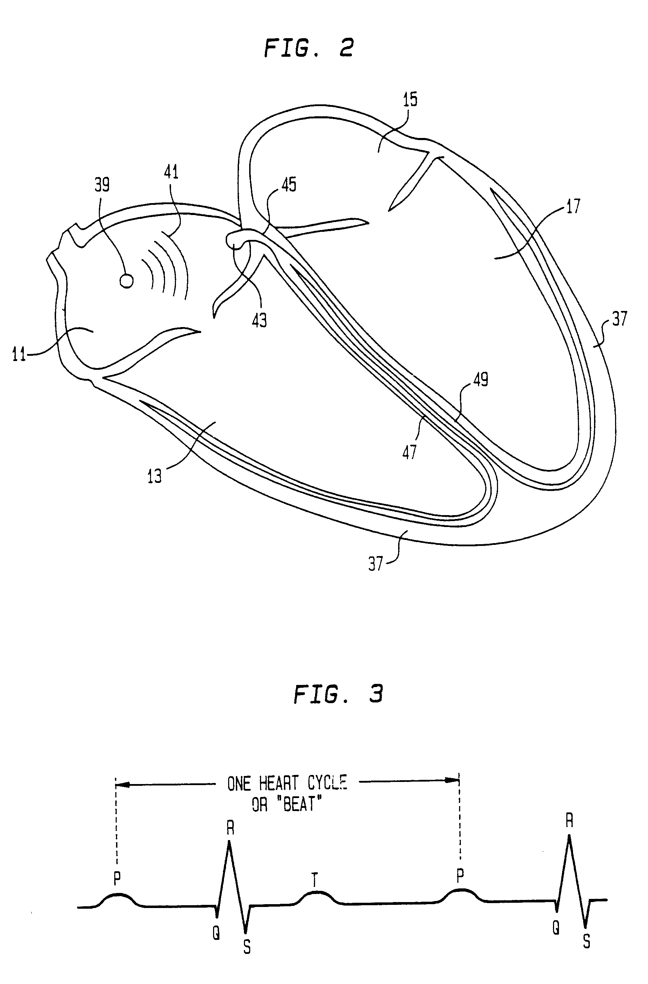

Fig. 2∅ is a partially cut-away atrial electrode of the type utilized in the embodiment of Fig. 1∅;

The following additional drawings are presented in conjunction with that portion of the description designated as Appendix A, wherein:

Fig. A-1 is a block diagram of an alternative emobiment of the invention described in Appendix A;

Fig. A-2 is a logic diagram of the state or programmed timer 404 of Fig. A-1;

Fig. A-3 depicts a preferred realization of the demultiplexer 414 of Fig. A-1;

Figs. A-4 and A-5 illustrate a logic diagram of the state signal generator 406 of Fig. A-1; and

Fig. A-6 is a flow diagram of a representative program used to control the microprocessor 408 of Fig. A-1.

## DETAILED DESCRIPTION OF THE INVENTION

The following description is of the best presently contemplated mode of carrying out the invention. This description is not to be taken in a limiting sense but is made merely for the purpose of describing the general principles of the invention. The scope of the invention should be determined with reference to the attached claims.

Before describing the present invention in detail, it will be instructive to briefly review some fundamental operating principles associated with pacemakers, especially dual-chamber demand-type pacemakers. To best understand the operations of such pacemakers, it is helpful to first have a basic understanding of cardiac anatomy. Accordingly, reference is made to Fig. 1 wherein is shown a schematic representation of the heart and the flow of blood therethrough. The heart is essentially made up of four (4) chambers; a right atrium 11, a right ventricle 13, a left atrium 15, and a left ventricle 17. The atrium chambers function primarily as reservoirs into which incoming blood is received, while the ventricles function primarily as pumping chambers to pump the blood away from the heart to a specific destination. Blood, carrying carbon dioxide waste from the body cells, enters the right atrium by way of the superior vena cava 19 or the inferior vena cava 21. At the appropriate time, the right atrium 11 contracts and pushes the blood through the tricuspid valve 23 into the right ventricle 13. A short time later, the right ventricle 13 contracts and pushes or pumps the blood through the pulmonary valve 25, which valve leads to the pulmonary artery 27. The pulmonary artery 27 divides into two branches, one leading to the right lung and the other leading to the left lung. At the lungs, the carbon dioxide in the blood is removed and replaced with fresh oxygen. The oxygenated blood returns from the lungs in the pulmonary vein 29, also divided into two branches, one branch for each lung, and is deposited in the left atrium 15. At approximately the same time that the right atrium 11 is contracting, the left atrium 15 also contracts and pushes the blood through the mitral valve 31 into the left ventricle 17. The left ventricle 17 contracts at approximately the same time as the right ventricle contracts and pushes or pumps the blood through the aortic valve 33 into the aorta 35. The aorta is the main artery that delivers the blood throughout the body. The natural rhythm of the heart thus includes the contraction of the atria, followed a short time later by the contraction of the ventricles. The ventricles do most of the work of the heart, as evidenced by the thickness of the heart muscle or myocardium 37 that surrounds both the right and left ventricles 13 and 17.

Referring next to Fig. 2, there is shown a simplified diagram of the heart showing the four (4) chambers thereof. For the sake of clarity, many of the elements associated with the heart have been omitted from the drawing of Fig. 2. Located in the right atrium 11 is an S-A node 39. The S-A node is often referred to as the heart's natural pacemaker. This is because the S-A node 39 begins the electrical impulse, depicted in Fig. 2 as the wavefront 41, that spreads in wave fashion to stimulate both the right atrium 11 and the left atrium 15. It is this electrical impulse 41 that causes the depolarization of the muscle tissue that forms the walls of the atria, thereby causing atrial contraction to occur. Also included in the right atrium is an A-V node 43. The A-V node 43 is stimulated by the electrical impulse 41 propagated from the S-A node 39. Upon stimulation, and after a short pause (typically about 0.1 seconds), the A-V node initiates an electrical impulse that starts traveling down an A-V bundle 45. After a short distance the A-V bundle 45 divides into a right bundle branch 47 and a left bundle branch 49. These left and right bundle branches distribute the electrical impulse throughout the myocardium or heart muscle 37, thereby causing the ventricles to depolarize and contract.

Shown in Fig. 3 is a representation of the various waveforms that are generated, as sensed by skin electrodes placed on the chest, in response to the above-described activities. A P-wave represents the depolarization of both atria. The QRS-wave, commonly referred as the QRS complex, represents the electrical impulse as it travels from the A-V node to the various fibers branching from the left and right bundle branches 47 and 49 as it is distributed into the myocardial cells, thereby causing ventricular depolarization. The T-wave represents the repolarization of the ventricles so that they may be stimulated again. (Note, repolarization of the atrium is usually not sensed because it occurs about the same time as the QRS complex, and any signals representative of atrial repolarization are therefore masked out by the QRS complex.) One cardiac cycle is represented by a P-wave, a QRS complex, and a T-wave. This cycle is repeated continuously as the heart pumps the blood as described in connection with Fig. 1. In summary, the P-wave represents depolarization of the atria. The QRS complex, sometimes referred to as simply an R-wave, represents the depolarization of the ventricles. Depolarization/contraction of the atria, followed a short time thereafter by depolarization/contraction of the ventricles, are the cardiac events that must occur if the heart is to efficiently perform its function as a pump in distributing blood throughout the body.

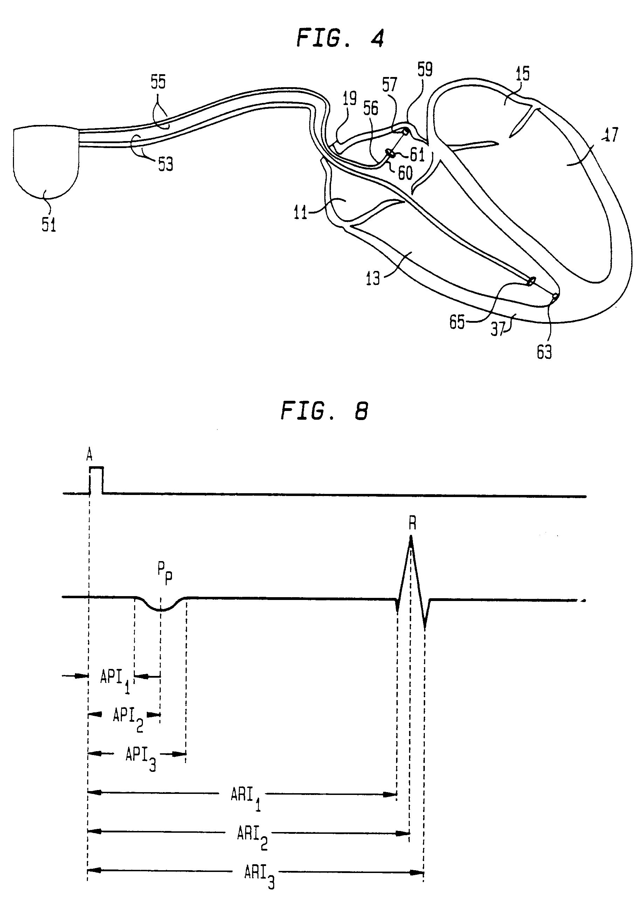

Referring next to Fig. 4, there is shown a simplified representation of one way that an implanted pacemaker 51 may make electrical contact with the heart. Fig. 4 depicts the use of two (2) bipolar leads 53 and 55, each being directed into a separate chamber of the right heart. A bipolar lead comprises a single filar that includes two (2) electrically insulated conductors. For example, the lead 55 includes a first conductor 56 that is electrically connected to a distal tip 57 of the lead. This distal tip is typically placed in a cavity of the right atrium referred to as the atrial appendage 59. A known distance from the distal tip 57, an electrode ring 61 is electrically connected to the other conductor 60 of the bipolar lead 55. Similarly, a distal tip 63 and a conductive ring 65 are associated with the bipolar lead 53 that is placed in the apex of the right ventricle 13. The manner in which the leads 55 and 53 are inserted into the heart, as well as the manner in which the pacemaker 51 is implanted in the body of a patient, are well known in the art.

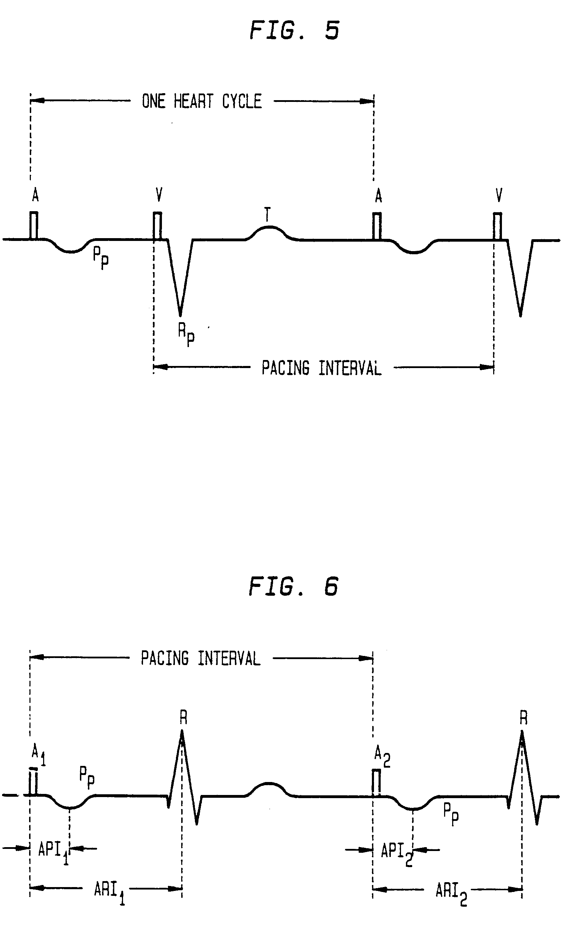

Fig. 5, shows a timing diagram that illustrates the response of the heart to stimulation pulses that are generated by an implanted pacemaker, such as the pacemaker 51 shown in Fig. 4. In response to an atrium stimulation pulse, or A-pulse, delivered to the right atrium 11 through the distal tip 57 of lead 55 (Fig. 4), both atria contract and a P-wave is generated. Because the stimulating A-pulse originates from a different point within the right atrium than does the normal stimulating pulse from the S-A node 39 (Fig. 2), the P-wave generated in response to this A-pulse does not appear the same as a naturally occurring P-wave. For purposes of this application, this difference between a P-wave in response to an A-pulse and a P-wave in response to the naturally occurring pulse from the S-A node is depicted as a P-wave of opposite polarity. The waveform of Fig. 5 is further distinguished by referring to it as the $P_P$- wave, indicating that it is a paced P-wave, or a P-wave in response to a pacing signal. Similarly, in response to a stimulation pulse applied to the right ventricle, an R-wave is generated, represented in Fig. 5 as an inverted $R_P$ pulse. The R-wave in Fig. 5 is shown inverted from the R-wave shown in Fig. 3 because the stimulating pulse propagates through the ventricle chamber in a different direction than does the natural stimulating pulse that propagates through the left and right bundle branches. Hence, for purposes of this application, the natural responses or natural depolarizations of the heart are represented in the figures as a positive P-wave (a waveform going in the upwards direction) and a positive R-wave. Depolarizations of the atria or ventricles in response to an externally generated stimulation pulse, such as occurs with a pacemaker, are represented as a negative going $P_P$ wave or $R_P$ wave.

With reference to Fig. 6, one possible response to an atrium stimulation pulse, A, is shown. As is seen in Fig. 6, in response to the pulse $A_1$, a $P_P$ wave form is generated a short time later, which time interval is identified as $API_1$ (referring to the first A-P interval). In response to the atria depolarization evidenced by the $P_P$ wave, and in the absence of A-V block, the ventricles depolarize and contract without the need of a stimulation pulse. Such depolarization occurs a time $ARI_1$ later (referrring to the first A-R interval of the sequence shown in Fig. 6). At an appropriate time subsequent to the generation of the first atrium stimulation pulse $A_1$, a second atrium stimulation pulse, $A_2$, is generated by the pacemaker. In response to the $A_2$ stimulus, a second $P_P$ wave is generated a time $API_2$ after the generation of the $A_2$ pulse. Again, a naturally occuring R-wave occurs a time $ARI_2$ subsequent to the generation of the A2 pulse. The AP/AR intervals shown in Fig. 6, designated as $API_i$ and $ARI_i$, are time intervals that play a key role in connection with the preferred embodiment of the invention described herein. More particularly, it is changes in these time intervals, when sensed after monitoring the individual time intervals over a plurality of heart cycles, that indicate changes in the physiological need of the body within which the heart is located. Hence, this particular embodiment of the invention is concerned with measuring the AP/AR intervals, and in processing these measured intervals in such a fashion that the resulting processed mearsurement can be used to adjust the control parameters of the pacemaker in order to change the pacing rate thereof so that this pacing rate approxiamtes the changes that would occur in a healthy (non-paced) heart in response to the same changes in physiological need.

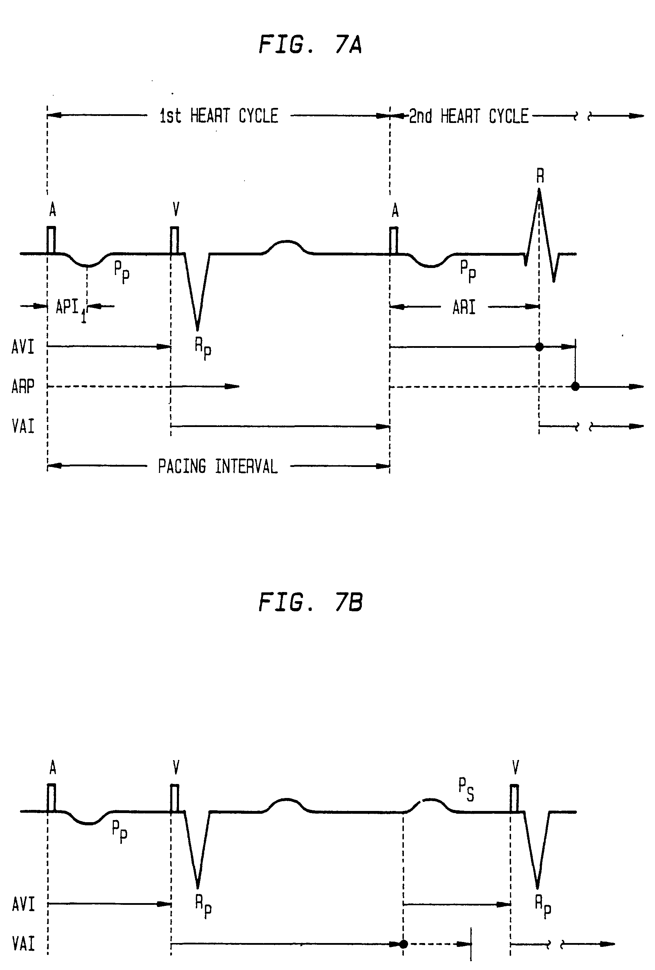

Referring next to Figs. 7A and 7B there are shown further timing diagrams that define various intervals that are commonly used in controlling a dual-chamber demand-type pacemaker. The description that follows is somewhat simplified, but will be useful in understanding the operation of a dual-chamber pacemaker. Additional details associated woth a preferred pacemaker are described hereinafter in connection with Figs. 14 and 15, and in Appendix B. In a demand-type pacemaker it is common to define an

6

escape interval during which activity within the heart is sensed. If a natural cardiac event occurs during this escape interval, that is if a natural P-wave or R-wave is sensed, then a corrresponding stimulating pulse need not be generated. Not only does this mode of operation allow the heart to function in its natural state, if it is able, but it also helps conserve the limited power stored within the battery of the pacemaker. In Fig. 7A, it is seen that both the AP interval and AR interval are illustrated as in Fig. 6. Also shown in Fig. 7A, however, is an AVI, or AV interval. This is a prescribed time set by the pacemaker during which a naturally occuring R pulse must occur, if one is to occur, prior to the generation of a ventricle stimulation pulse, V. As indicated in Fig. 7A, the AV interval has timed out for the first heart cycle shown, thereby causing the V-pulse to be generated. During the second heart cycle, however, the AV interval has not yet timed out at the point in time when the naturally occurring R-wave appears. Thus, there is no need for the pacemaker to generate a V stimulation pulse during the second heart cycle. Also illustrated in Fig. 7A is an atrial refractory period, or ARP. During this refractory period, the normal sensing mechanisms used within the atrium are nonresponsive. This refractory period is analogous to the natural refractory period of myocardial tissue immediately following depolarization and prevents the pacemaker from detecting any depolarization signals or noise that might result in timing errors. The refractory period is made up of two components, the absolute refractory period (indicated by the dashed line), during which detection of all signals is blocked, and a noise sampling or relative refractory period (represented by the solid line) during which detected signals are evaluated for a repetitive rate. As will be evident from the discussion that follows, the atrial refractory period, or ARP, does not prevent the detection of a $P_P$ pulse because, as previously stated, this pulse is detected using a sensing means different from the normal atrial sensing probe.

Also shown in Fig. 7A is a VA interval, or VAI. The beginning of this interval is initiated by the generation of a V-stimulation pulse, or the sensing of a natural R-wave. This VA interval, less the ARP, defines the time during which a natural (non-paced) P-wave must be detected if the A stimulation pulse is to be inhibited. As is evident from Fig. 7A, the pacing interval or rate set by the pacemaker is equal to the VA interval, VAI, plus the AV interval, AVI. Hence, by varying or adjusting these two time periods, the pacing interval of the pacemaker can be controlled, thereby controlling the heart rate.

Referring next to Fig. 7B, a different cardiac event sequence is illustrated. In this figure, it is seen that an A-pulse, or atrial stimulus, is first generated, causing a $P_P$ wave (or atrial depolarization) to occur. The AV interval is initiated by the generation of the A-pulse. At the conclusion of the AV interval, a V-pulse or ventricle stimulation pulse is generated because no natural occuring R-wave was sensed prior to that time. In response to the generation of the V-pulse, the ventricle depolarizes as evidenced by the $R_P$-wave, and the next VA interval is initiated. Before the VA interval, or VAI, terminates, however, a natural P-wave (identified as P, and sometimes referred to as a sinus P-wave) occurs. Accordingly, there is no need for the pacemaker to generate an atrium stimulation pulse. The sensing of the P wave re-initiates the AV interval. During this interval, the sensors in the ventricle are monitoring the ventricle activity to determine if a naturally occuring R-wave is present. For the situation shown in Fig. 7B, a naturally occurring R-wave does not occur prior to the termination of the AVI, So a V-pulse is generated, thereby causing a paced $R_p$ wave to occur, indicating ventricular contraction.

It is to be understood that Figs. 7A and 7B represent simplified timing diagrams that illustrate only two of a very large number of heart event sequences that can occur. Volumes have been written by those skilled in the art describing the various heart rhythms, and abnormalities related thereto, that may occur. While most modern pacemakers are designed to recognize and deal with many of these abnormalities, a description of such matters herein would add little to the understanding of the present invention. In fact, a detailed description of all the heart rhythms and abnormalities associated therewith could obfuscate an understanding of the present invention. Accordingly, no such detailed description will be presented herein beyond that which is believed necessary to fully understand the present invention.

At this point it would be helpful, however, to review the different type of pacemakers that are available, and with which the present invention could be used. Generally, pacemakers are identified by a three letter code. The first letter represents the chamber of the heart that is paced. This letter may be a V for ventricle, an A for atrium, or a D for double (meaning that both the ventricle and atrium are paced). The second letter indicates the chamber sensed. Again the possible letters used are the V for ventricle, the A for atrium, a D for double, or the number "∅" for none. The third letter indicates the mode of response of the pacemaker. A "T" indicates a triggered mode of response wherein the pacemaker regularly sends a stimulation pulse to the heart. An" I" indicates an inhibited mode of response, indicating that a stimulation pulse will be delivered to the heart unless it is inhibited by a naturally occurring cardiac-event that occurs within a predefined time interval. A "D" indicates a double mode of response, wherein the pacemaker may either operate in a triggered or inhibited mode. It is contemplated that the third letter could also be used to indicate the addition of the physiological sensor of the present invention to the pacemaker. For example, if the third letter were a "P", for "physiological", then that could be used to signal a multimode response pacemaker that includes automatic pacing interval adjustments in response to the sensed change in physiological neeed. Hence, a VVP pacer could be one in which the ventricular chamber is paced, the ventricular chamber is sensed, and the V-R interval is measured and used as a controlling parameter to automatically adjust the pacing interval in accordance with the teachings of the present invention. A DDP pacemaker, in accordance with this marking scheme, would be the most versatile of all modern pacemakers. This is because such a pacemaker could not only be programmed to operate in any mode that is best suited for the particular patient, but it would also automatically adjust the pacing interval in accordance with the sensed physiological need of the patient, regardless of the chamber or chambers of the heart that are being paced or sensed. While there are several DDD pacemakers currently available on the market, such as the AFPII 283 manufactured by Pacesetter Systems, Inc. of Sylmar, California, none are yet available that include the sensing and adjustment capabilities of the present invention. However, it is to be understood that the present invention -- a physiological sensor that can be used to automatically adjust the pacing rate delivered or controlled by a pacemaker-- could be adapted for use with any of the existing or yet to be designed pacemakers.

There are essentially three operating modes or types of pacemakers that are presently envisioned for use with the physiological sensor of the present invention. These are:

1. A single chamber atrial pacemaker;

2. A single chamber ventricular pacemaker; and

3. A dual-chamber pacemaker.

A single chamber atrial pacemaker would measure the A-P interval and use this measurement to adjust the pacing interval in an appropriate direction. A single chamber ventricular pacemaker would measure the V-R interval and use this measurement to adjust the pacing interval in an appropriate direction. A dual chamber pacemaker could measure either the A-P interval, the V-R interval, or the A-R interval, depending upon its mode of operation, and use these measurements to adjust the pacing interval in an appropriate direction. Because the dual chamber pacemaker is the most versatile, and because the single chamber pacemakers are really subsets of the dual chamber pacemaker (at least insofar as an understanding of the present invention is concerned), the description that follows is directed towards a dual chamber pacemaker. However, it is to be emphasized that the present invention is not so limited. Moreover, where the description given hereinafter refers to the measurement of the A-P interval, it is to be understood that these same teachings could be applied in measuring or describing the V-R interval.

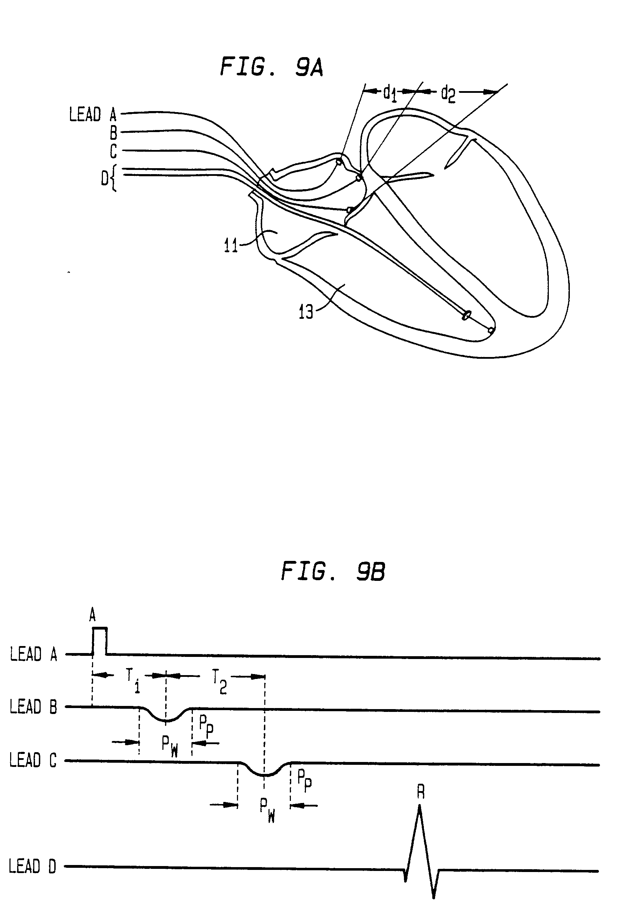

Referring next to Figure 8, there is shown a timing diagram having an expanded time base illustrating some variations of the A-P and A-R time intervals that could be utilized with the present invention. Because the paced P-wave is not a sharp pulse as are the stimulation pulses, such as the A-pulse, it may be advantageous to terminate the A-P interval at various points on the $P_P$ wave. For example, as illustrated in Figure 8, the API could terminate at the commencement of the paced P-wave, at the peak of the paced P-wave, or at the conclusion of the paced P-wave. As a practical matter, the detection circuitry is the simpliest and less costly if the peak of the P-wave is used as the detection point. Moreover, because there may be variations in the sensing circuitry, the peak of the P-wave may not be consistently sensed, but some threshold on the P-wave will be sensed with sufficient consistency for a meaningful API measurement to be made. Likewise, the ARI measurement will typically be made to the peak of the R-wave, designated as $ARI_2$ in Fig. 8, because this is the easiest signal to detect. However, assuming that appropriate detection circuitry is available, the leading edge, designated $ARI_1$, or the trailing edge, designated $ARI_3$, could be used in place of the peak ARI measurement. Similarly, a detection circuit that senses the maximum slew rate of the R-wave could be employed.

Next, referring to Fig. 9A, there is shown an alternative embodiment of lead placement within the heart that could be used with the present invention. In accordance with this embodiment, a plurality of unipolar leads, identified as leads A, B, and C, may be selectively placed within the atrium 11 of the heart. A conventional bipolar lead, identified as lead D, is shown as being inserted in the ventricle 13 of the heart. In accordance with the embodiment shown in Fig. 9A, it is contemplated that the atrium stimulation pulse, or A-pulse, would be delivered to the heart through lead A. The tip of lead B, being spaced a fixed distance from lead A, would sense the generation of the P-wave at a certain time later, identified as $T_1$ in the timing diagram of Fig. 9B. The time $T_1$ is a function of the propagation rate of the stimulation pulse as this stimulation pulse travels the distance $d_1$ in Fig. 9A. As described thus far, it is noted that the tip of lead B in Fig. 9A is performing the same function as the ring electrode 61 of the bipolar lead 55 in Fig. 4. A third lead, lead C, could also be employed, with its tip spaced a distance $d_2$ from the tip of lead B. Hence, as indicated in Fig. 9B, the $P_p$ wave sensed by lead C would be delayed by an amount equivalent to the propagation delay time of the stimulation pulse through the atrium as it travels the distance $d_2$. It is within

the scope of the present invention that either the times $T_1$, $T_2$, $T_1$ - $T_2$, or $T_1$ + $T_2$, could be used as the timing interval that is measured in order to determine changes in physiological need in accordance with the present invention. It is also contemplated that changes in the width of the paced P-pulse, PW, could be used to indicate physiological need. Further, while Fig. 9A illustrates unipolar leads placed in the atrium, and a bipolar lead placed in the ventricle, it is to be understood that other combinations of unipolar/bipolar leads, all unipolar leads, or all bipolar leads (Fig. 4) could be employed. Further, a tripolar lead having at least two spaced-apart ring electrodes in addition to a distal tip electrode could be used to achieve the function described above.

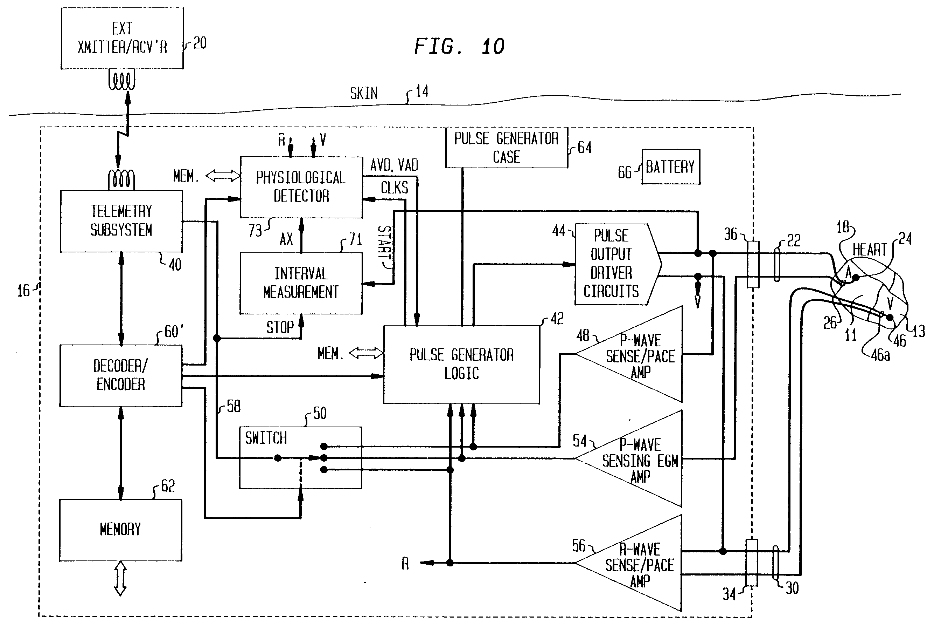

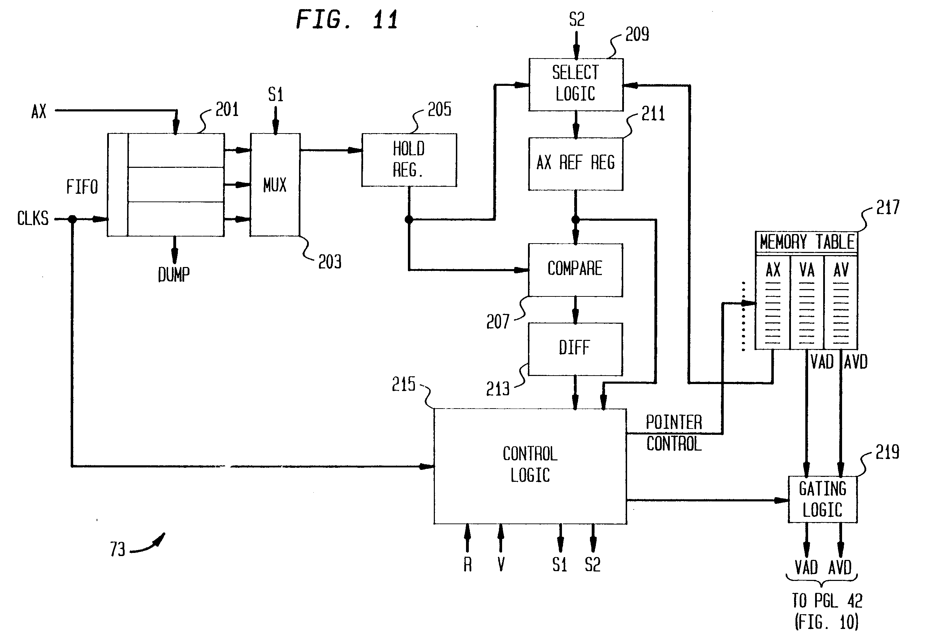

Referring now to Figure 10, a block diagram of an implanted pacemaker 16 according to the invention is shown, the pacemaker 16 being connected to a user's heart 18. At appropriate times, the pacemaker 16 my be electromagnetically in contact with a telemetry transmitter and receiver 20 external to the user's skin 21. A conventional bipolar atrial lead 22 is provided having a first or tip electrode 24 at its distal end and a second electrode 26 spaced apart from the tip electrode 24 and in the configuration of a typical bipolar lead ring electrode. It may be understood that a second ring electrode and an associated amplifier may be used for greater signal strength in sensing the electrical activity in the atrium. The tip electrode 24 is located in contact with atrial tissue of the heart atrium 11. A bipolar ventricle lead 30 is located in the heart ventricle 13 and is attached to the pacemaker 16 through a ventricular connector 34. Of course, a unipolar ventricle lead could also be used. The atrial lead 22 is connected to the pacemaker 16 through an atrial connector 36. The pacemaker 16 includes a telemetry subsystem 40 for transmitting data and parameter values to the external telemetry transmitter and receiver 20, and for receiving data instructions and the like from the external telemetry transmitter and receiver 20. The pacemaker 16 also includes pulse generator logic circuitry 42 which, in turn, controls pulse output driver circuits 44 for providing both atrial and ventricle stimulation pulses. The atrial output of the pulse output driver circuits 44 is connected through the atrial connector 36 to the atrial tip electrode 24 for stimulation of the atrium; the ventricle output of the pulse output circuits 44 is connected through the ventricle connector 34 to a ventricle tip electrode 46 for stimulation of the ventricle. A P-wave sense/pace amplifier 48 having bandpass characteristics as explained below is also connected through the atrial connector 36 to the atrial tip electrode 24 for receiving electrical signals present at the electrode 24. The output of the P-wave sense/pace amplifier 48 is also connected to the pulse generator logic circuitry 42 and to switch 50, the purpose of which will be explained below. The implanted pacemaker, in operating as a "demand" type pacer, would not provide stimulation to the atrium when amplifier 48 provides at its output a signal indicating the sensing of an intrinsic or sinus P-wave. A second amplifier, a P-wave sensing EGM amplifier 54 having bandpass characteristics as explained below has its input connected through the atrial connector 36 to the second atrial electrode 26. The output of the P-wave sensing amplifier is also connected to the switch 50. An R-wave sense/pace amplifier 56 is also provided, its input being connected to the pulse output driver circuits 44, the ventricle tip electrode 46, and a ventricle ring electrode 46a, these last two connections being made through the ventricle connector 34. The output of the R-wave sense/pace amplifier 56 is connected to the pulse generator logic circuitry 42 for inhibiting a ventricle stimulation pulse in the presense of spontaneous ventricular activity, (i.e., in the presence of a naturally occuring, non-paced, R-wave) and to the switch 50. Amplifier 56 has a sufficiently broad band-pass characteristics to pass electrical signals of substantialy all native (intrinsic) ventricular activity. The output of the switch 50 is connected via a line 58 to the telemetry subsystem 40 for real time transmission of the output of either the P-wave sense/pace amplifier 48, the P-wave sensing amplifier 54 or the R-wave sense/pace amplifier 56. The specific amplifier output to be transmitted is selected by the physician via instructions transmitted by the external telemetry transmitter and receiver 20 and received by the implanted telemetry subsystem 40. These instructions are decoded by a decoder and encoder 60'. The output of the decoder and encoder 60' is utilized to establish which amplifier output 48, 54 or 56 is to be connected to the telemetry system 40 for transmission to the external telemetry transmitter and receiver 20. Although the switch 50 is shown as a switch, it should be readily apparent that any kind of selectable connecting means could be employed to provide continuity between one of the amplifiers 48, 54 and 56 and the line 58. Further, two or more of the amplifier outputs could be transmitted simultaneously if proper provisions were made within the telemetry subsystem 20. In addition, a memory 62 is provided which receives parameter information from the decoder and encoder 60', this parameter information being utilized to control the pulse generator logic circuitry 42. The tip electrode 24 for stimulating the atrium and the tip electrode 46 for stimulating the ventricle may be utilized in a unipolar configuration with the return path being provided through a conductive portion of the pulse generator case 64 which is connected to the pulse output driver circuits 44. Alternatively, bipolar operation may be employed where the return path is through the conductor connected to the ring electrode, although such bipolar operation may make sensing of a $P_p$-wave difficult, as explained below. A battery 66 is also incorporated for providing power to the implanted pacemaker 16. It should also be recognized that although an implanted pacemaker is shown for illustrative purposes, the invention is in no way limited to an implanted pacemaker. An external pacemaker could also be provided in accordance with the teachings of the invention. Further, although bipolar atrial and ventricular leads were chosen, for illustrative purposes, a unipolar, ventricular lead could also have been utilized provided appropriate connectors were available on the pacemaker, and a plurality of unipolar leads could have been used within the atrium as shown in Fig. 9. Similarly, a multi-conductor atrial lead could be provided with two of the conductors providing a bipolar atrial lead and the third conductor being connected to the P-wave sensing ECG amplifier 54 shown in Figure 10.

Also included in the pacemaker 16 is an interval measurement circuit 71 and a physiological detector 73. The interval measurement circuit 71 comprises an appropriate time interval measurement circuit. The time interval measured is started by the generation of an atrium stimulation pulse as generated by the pulse output driver circuits 44, and is stopped by the output of either the P-wave sensing EGM amplifier 54 (which output indicates the sensing of a $P_p$ wave), or the output of the R-wave Sense/Pace amplifier (which output includes the sensing of an intrinsic R-wave) The interval measurement circuit therefore measures the ARI or the API, as selected by control signals received through the telemetry subsystem. Further, it is contemplated that one mode of operation could include always measuring the ARI, if present, but if not present, as for example in a situation where heart block exists, then automatically reverting to measuring the API.

The time interval measured by the interval measurement circuit 71 is passed to the physiologcal detector 73, which detector 73 processes the measured interval as described more fully below and generates appropriate control parameters as a result of this processing that are delivered back to the pulse generator logic 42.

The manner in which the implanted pacemaker 16 operates will now be explained. This explanation will be given in two parts, a first part of which relates to the sensing function of the pacemaker, and a second part of which relates to the physiological detecting function of the pacemaker and the manner in which the pacing rate is varied or controlled as a result of this physiological detection. Because the telemetry functions and pulse generator/pulse delivery functions are conventional functions performed by pacemakers known in the art, no further explanation of these functions will be presented herein.

Sensing Function

First, with reference to the sensing function of the pacemaker, operation of the pacemaker 16 shown in Fig. 10 can be best understood by refrence to Figures 16A, 16B, 17A and 17B. One of the problems associated with atrial pacing is determining whether atrial or P-wave capture has been effected by atrial stimulation pulses. This involves sensing the occurrence of a paced $P_p$ wave. In prior art systems, the sensing circuit corresponding to the P-wave sense/pace amplifier 48 in Figure 10 sensed signals present at the electrode at the lead distal end (corresponding to the tip electrode 24 in Figure 10). Referring now to Figure 16A, the voltage present at the output of the P-wave sense/pace amplifier 48 in the presence of an atrial stimulation pulse corresponds in general to the waveform shown at 70. Thus, the output of the P-wave sense/pace amplifier 48 is saturated during the period "S" shown in Figure 16A. Because the paced $P_p$-wave voltage is small with respect to the saturation voltage caused by the A stimulation pulse, which $P_P$-wave is represented in Figure 16A by the wave 72, it is difficult, if not impossible, to pick out the time at which the $P_p$ wave occurres relative to the stimulation pulse A, which A-pulses occur at the times indicated by the arrows 74. Because of this difficulty in determining when the $P_p$-wave 72 actually occurred relative to stimulation pulse occurrence as shown at 74, it is difficult for the physician to determine if the stimulation pulse has effected P-wave capture. It is also difficult, if not impossible, to measure the API as is required by the present invention.

Referring now to Figure 16B, a simplified representation of the heart is shown, showing a pacemaker 76 according to the invention, having a conventional atrial lead 78 and, also having a stimulation and sensing electrode 80 at its distal end and a second or $P_p$-wave sensing electrode 82 spaced apart from the stimulation electrode 80. By way of example only, the atrial lead 78 is configured in the form of a J at its distal end so that the stimulation electrode 80 can be located within the atrial appendage (not shown). The heart sinus node 84 is also shown, as well as a ventricle lead 86 having its stimulation electrode 88 located in the ventricular apex. It can be appreciated that the further the sensing electrode 82 is spaced-apart from the stimulation electrode 80, the less the stimulation pulses will interfere with $P_p$-wave sensing by the sensing electrode 82. This is because the electrical stimulation signal, by the time it propagates to the sensing electrode 82, has decreased in amplitude a sufficient amount to preclude it from interfering with the sensing of the $P_P$ wave. However, it should be apparent that the sensing electrode 82 cannot be so far removed from the stimulation electrode 8∅ that it would no longer be within the heart atrium. For the embodiment shown, all electrodes 8∅, 82 and 88 use the case of the pacemaker 76 as a return electrode, the case being positive with respect to a negative going pulse present at both stimulation electrodes 8∅ and 88. Another advantage of utilizing the spaced-apart sensing electrode 82 for P$_p$-wave detection is that the P-wave electrical characteristics as picked up by the sensing electrode 82 differ because of the direction of propagation. This is shown by the arrows 9∅ and 92, arrow 9∅ showing the propagation direction from the stimulation electrode 8∅ and arrow 92 showing the propagation direction from the sinus node 84. As explained previously, this propagation direction is what causes the polarity of the two signals to be different. Further, in determining P-wave capture, this allows the physician to determine if the P-wave occurred as a result of spontaneous atrial activity or stimulated atrial activity. Moreover, because of the different distances between the sensing electrode 82, the sinus node 84, and the stimulation electrode 8∅, it can be appreciated that even if the sinus node 84 is operating in synchronism with the stimulation pulses, the known propagation time between a stimulation pulse and P-wave generation could be used to determine if P-wave generation were due to spontaneous or stimulated atrial activity. Further, it can be appreciated that although a typical bipolar atrial electrode 78 is utilized, all three electrodes 8∅, 82 and 88 operate in a unipolar manner in that they all use the pacemaker 76 case as a common return electrode. Alternatively, the ventricular lead 86 could be a bipolar lead, and the sensing/pacing in the ventricle could be operated in a bipolar manner without interfering with the P-wave detection in the atrium.

Detection of atrial capture can be further understood in reference to Figures 17A and 17B, which figures show actual intercardial electrograms as sensed by an implanted pacemaker and transmitted to a suitable display device. Referring to Figure 17A, atrial stimulation pulses 1∅∅ can be seen. Further, P-waves 1∅4 and R-waves 1∅6 can also be seen. The time differential D between atrial stimulation and P-wave occurrence in successive cycles can be seen to be constant. Thus, the physician can assume that P-wave capture as a result of the atrial stimulation pulses has occurred provided that the distance D corresponds approximately to the propagation delay due to the distance between the stimulation electrode 8∅ and the sensing electrode 82 as explained in conjunction with Figure 16B. Referring now to Figure 17B, the time differentials D' and D'' between the atrial stimulation pulses 1∅∅ and P-wave occurrences 1∅2 can be seen to be different. Thus, the physician can conclude that P-wave capture by the atrial stimulation signals has not occurred but that the P-waves are spontaneous or "native" or "intrinsic" in origin. Under normal circumstances with respect to Figure 17B, the physician would assume that the magnitude of the stimulation pulses is below the stimulation threshold of the particular patient's atrium and would accordingly increase their magnitude until P-wave capture occurred, that is, until an EGM signal similar to that shown in Figure 17A is observed. Again, in prior art systems, it would be impossible to observe the presence of P-waves utilizing the stimulation electrode 8∅ (Fig. 16B) as the sensing electrode due to the saturation of the P-wave sense/pace amplifier resulting from the atrial stimulation pulse (see Fig. 16A).

Figure 18 shows a simplified block diagram of a typical P-wave or R-wave wave amplifier 1∅9 such as those shown in Figure 1∅ as blocks 48, 54 and 56. The amplifier 1∅9 includes an amplification portion 11∅ and an input filter 112. The difference between the P-wave sense/pace amplifier 48 and the P-wave sensing amplifier 54 is in the bandpass characterisitics of the amplification portion 11∅ and filter 112 combination. The amplitude and bandpass characteristics of the P-wave sense/pace amplifier 48 are chosen to provide to the pulse generator logic circuitry 42 a positive indication of P-wave occurrence in the absence of an atrial stimulation pulse, while at the same time rejecting non-P-wave signals such as far-field R-wave signals and muscle electrical noise. This is to allow the pulse generator logic circuitry 42 to determine if the atrium is operating spontaneously or whether an atrial stimulation pulse is required. It is the output of this amplifier 48 that is subjected to the atrial refractory period discussed previously.

The purpose of the P-wave sensing amplifier 54 (Fig. 1∅) is to provide an electrogram of all, or most all, atrial electrical activity including an indication of P$_p$-waveoccurrence in the presence of an atrial stimulation pulse. Thus, the precise characteristics of the P$_p$-wave and its location with respect to an atrial stimulation pulse must be determinable in order to measure the AP interval and in order to determine if the P-wave is occurring spontaneously or is occurring as a result of an atrial stimulation pulse.

In order to meet these different requirements, the amplfier 11∅ and filter 112 combination of the P-wave sense/pace amplifier 48 as shown in Figure 1∅ can be chosen to have a center frequency at 6∅Hz and 3db points at approximately 1∅Hz and 1∅∅Hz. The purpose of this U-shaped frequency response is to maximize detection of the intrinsic P-wave which has a large frequency component near 6∅Hz and to reject other signals such as some of that from the heart R-wave which by the time it reaches the atrium has lower frequency components and muscle electrical noise which has mostly higher frequency components. Thus, the bandpass characteristics of the P-wave sense/pace amplifier 48 must be chosen to attenuate all electrical signals within the atrium other than the frequencies that most characterize the intrinsic P-wave. Of course the bandpass characteristics described above are only representative of one embodiment, and other response characteristics could be chosen. For example, a response curve having a peak of between 4Ø Hz and 8Ø Hz and the 3db points could lie between Ø.1Hz and 5ØØ Hz could be chosen. The teaching of the invention merely is that the P-wave sense/pace amplifier 48 be chosen to pass signals characteristic of the intrinsic P-wave while tending to reject signals that are not characteristic of the intrinsic or sinus P-wave. Thus peak detection circuitry in the pulse generator logic circuitry 42 (Fig. 1Ø) can be triggered by the output of the P-wave pace/sense amplifier 44 without danger of a false detection due to other electrical activity in the atrium.

The P-wave sensing EGM amplifier 54 is chosen to have a response that is essentially flat between about 3-1/2Hz and 2ØØ Hz. This is to allow the $P_p$-wave to be sensed and to allow the physician to see all electrical atrial activity for a complete understanding of the atrial electrical environment including any T-wave ventricle signals and any farfield R-wave signals that are present. However, the invention is in no way limited to a $P_p$-wave sensing EGM amplifier 48 having a flat response, and a frequency response such as that of the P-wave sense/pace amplifier could also be utilized. In Particular, use of such a frequency response that improves the ability to make the necessary AP interval measurement would be desired.

The above can be further understood by referring to Figure 19. Here the P-wave sense/pace amplifier response 113 and the P-wave sensing EGM amplifier response 114 can be seen. As can be seen, the response 113 is chosen to pass the P-wave frequency and attenuate the frequencies associated with other physiologic events as shown at 115 and 116 in order to provide a relatively high amplitude output corresponding only to R- and P-wave events. The response 114 is chosen to pass all frequencies in order to provide an accurate overall EGM signal to the physician. As shown, response 114, of the sensing amplifier includes T-wave ventricular frequencies and far-field signals. If inclusion of these signals makes it difficult to accurately measure the AP interval, then a narrower response, such as is indicated by the dashed-line response 115' could be employed.

Referring now to Figure 2Ø, a lead 22' of the type shown in Figure 1Ø as 22 is illustrated. Although a straight shank lead is shown for illustrative purposes, it should be recognized that a typical atrial-J lead could be utilized in the application shown in Figure 1Ø, and thus a portion of the distal end of the lead could be J-shaped. The lead 22' includes a tip electrode 24' which is connected through a spirally-wound conductor 117 to a first terminal 118. A ring electrode 26' is attached through a spirally-wound conductor 122 to a second terminal 12Ø, this conductor 122 being electrically isolated from the conductor 117 attached to the tip electrode 24'. The terminals 118 and 12Ø are adapted to connect to appropriate connectors in the pacemaker. Although the connector or terminal arrangement generally shown at 124 is a typical in-line type of connector, other connector arrangements could be utilized such as having each terminal coming out of the proximal end of the lead to form a Y-shaped connector. The ring electrode 26' is spaced apart from the tip electrode 24' a distance such that when the tip 24' is located in the atrial appendage, the ring electrode 26' will also be located within the atrium. As Previously explained, Figure 2Ø merely illustrates a typical bipolar atrial lead which is utilized in the Figure 1Ø embodiment while having its tip electrode and ring electrode operate in a unipolar fashion. Thus, an implantable pacemaker configured according to that shown in Figure 1Ø can be utilized with conventional bipolar atrial leads without requiring a special purpose lead to be utilized.

## Physiological Detecting Function

The second part of the manner in which the implanted pacemaker 16 (Fig. 1Ø) operates will now be explained. This part relates to the physiological detecting function of the pacemaker and how the pacing rate is varied or controlled as a result of this physiological detection. Referring back to Fig. 1Ø, the interval measurement circuit 71 can be realized using any suitable counting circuit that is started and stopped in the manner previously described. This counting circuit may be clocked by an appropriate high frequency signal (not shown) that is derived from the system clock used within the pulse generator logic 42. Once an interval measurement is made, this measurement is directed to the physiological detector 73.

Referring next to Fig. 11, there is shown a block diagram of a hardwave implementation of the physiological detector 73 of Fig. 1Ø. As an alternative, the function performed by the detector 73 shown in Fig. 11 could be realized using a microprocessor that is controlled by a program stored in ROM (read only memory) or in RAM (random access memory). An advantage of using RAM is that program optimization for a particular patient is possible using telemetric transfer of data via link 4Ø in Figure 1Ø. Such an alternative embodiment is briefly described in Appendix A attached hereto. As indicated previously, the interval measurement circuitry 71 of Fig. 1Ø measures the A-P interval, the A-R interval, or (for single chamber ventricular pacing) the V-R interval. For purposes of the discussion that follows in connection with Figures 11 and 12, this interval measurement will be generically referred to as the A-X interval measurement, where X refers to either the P (for the A-P interval measurement) or the R (for the A-R interval measurement), and where it is understood that the V-R measurement is processed similar to the processing of the A-X measurement, and where it is understood that a V-R measurement is included within the "A-X" designation for purposes of the description that follows.

The A-X interval measurement is loaded into a first-in, first-out, or FIFO register 201. The sequencing of the A-X value through FIFO 201 is controlled by clock signals, such as a clock corresponding to the pacing interval obtained from the pulse generator logic 42 (Fig. 10). Any signal that occurs during every heart cycle, such as the A-V delay, or AVD signal, could be used for this purpose. The FIFO 201 includes the capacity to hold at least three A-X interval measurements. Each measurement thus held is accessible through a multiplexer 203, the output of which is directed to a holding register 205. The contents of the holding register 205 may be directed to a comparision circuit 207 or through select logic 209 to an A-X reference register 211. The comparison, circuit 207 compares the contents of the holding register 205 with the contents of the A-X reference register 211 and sends the difference between the value stored in these two registers to a difference register 213. The contents of the difference register 213 are available to control logic 215, as are the contents of the A-X reference register 211. Also included as part of the physiological detector 73 is a memory table 217. While the memory table 217 is shown in Fig. 11 as actually being inside of the physiological detector 73, it is understood that this memory table could also be located within the memory circuits 62 shown in Fig. 10. Stored within the memory table are a range of values for the A-X interval. Corresponding to each of these stored values is a corresponding value for the V-A delay, or VAD, and the A-V delay, or AVD. As will be described below, the VAD and AVD comprise the principal timing elements of the AV and VA intervals, which intervals define a pacing interval. Accordingly, by adjusting the values of the VAD and AVD, the pacing interval delivered by the pacemaker to the heart can be controlled. The control logic 215 includes pointer control circuitry that allows it to scan the A-X values stored in the Memory Table 217. The stored value of A-X at any given point or location within the memory table 217 may be transferred through select logic 209 to the A-X reference register 211. Similarly, the VAD and AVD values corresponding to the A-X value that is addressed through the pointer control of the control logic 215 can be delivered through appropriate gating logic 219 to the pulse generator logic 42.

The operation of the detector 73 shown in Figure 11 is best understood with reference to the flow diagram of Figure 12A. This flow diagram defines the functions performed by the control logic 215. Referring to Fig. 12A, one of the first steps performed by the control logic 215 is to determine if the A-X interval measurement operating mode is enabled. This occurs at decision block 231. As will be discussed hereinafter, it is contemplated that other operating modes will be available with the pacemaker within which the present invention is utilized. Accordingly, if the A-X interval measurement is not enabled, then the pacemaker continues to operate in whatever other mode has been selected, as indicated in block 233 of Fig. 12A. If the A-X interval measurement has been enabled, then the next event that must occur for operation of the present invention is the sensing of an R-wave or a V-pulse, as indicated in processing block 235 of the flow diagram of Fig. 12A. (Note with reference to Fig. 10 that V and R inputs are shown going into the physiological detector 73. These inputs come from the pulse output driver circuits 44 and the R-wave sense/paced amplifier 56 respectively.) Once an R-wave or V-pulse has been sensed, then a determination is made as to whether three consecutive A-X intervals are present in the FIFO 201 (Fig. 11). This decision is indicated at decision block 237 of the flow diagram of Fig. 12. Should it be determined that three sequential A-X intervals are not present, then no further action is taken until the next R-wave or V-pulse is sensed. If, however, the FIFO 201 does contain three sequential A-X intervals therein, then each A-X interval, designated as $A-X_i$, is compared with the A-X value stored in A-X reference register 211. This A-X reference value is designated in Fig. 12 as $A-X_{Ref}$, and this comparison occurs at block 239 of the flow diagram of Fig. 12A. If this comparison indicates that all of the $AX_i$ values are greater than the A-X reference value, or if the comparision indicates that all of the $A-X_i$ values are less than the $A-X_{Ref}$ value, then an $AX_i$ trend has been established for purposes of the process shown in Fig. 12A. Determination of this trend, if present, is indicated at block 241 of the flow diagram of Fig. 12A. It is noted that if the trend is not present, then no further action is taken until the next R-wave or V-pulse is sensed, as indicated at block 235. If, however, a trend has been established at decision block 241 --that is, all of the values of $AX_i$ are either less than or greater than the value of $A-X_{Ref}$-- then a determination is made as to which $A-X_i$ value is closest to the $A-X_{Ref}$ value held in the A-X reference register 211 (Fig. 11), as indicated at block 243 of Fig. 12A. Once the $AX_i$ value closest to the $A-X_{Ref}$ value has been determined, this value is held in the holding register 205 while the pointer control of the control logic 215 begins to scan, the memory table 217. As indicated previously, if an A-X value stored in the Memory Table 217 is addressed by the pointer control of the control logic 215, this value may be transferred through the select logic 209 and held in the A-X

13

reference register 211. With the stored A-X value from the Memory Table 217 now in the A-X register 211, and the $AX_i$ value held in the holding register 205, the comparison circuitry 207 can again be used to determine the difference between the two values. If the values are not within a prescribed difference of each other, then the pointer control moves the table pointer in order to look for another value of A-X that is closer to the selected A value held in holding register 205. The steps of initiating scan of the Memory Table and moving the table pointer as above described are indicated at blocks 245 and 247, respectively, of the flow diagram of Fig. 12A.

Should the comparision of the stored A-X values and the selected $A-X_i$ value indicate a "match", as indicated at decision block 249 of Fig. 12A, (wherein "match" indicates that one value is within a prescribed difference of the other value, such as 2 percent), then the stored A-X reference value being held in the A-X reference register 211 remains held therein, as indicated at block 253 of Fig. 12A, and the corresponding AVD and VAD values at the current pointer address are transferred to the pulse generator logic 42, as indicated at block 255.

Because it is desirable that the pacing rate as controlled by the pacemaker not change too rapidly, a rate smoothing function is also employed in connection with the present invention. For purposes of this rate smoothing function, it is contemplated that the A-X values stored in the Memory Table 217 be stored in ascending or desending order. Thus, as the pointer control accesses various addresses within the Memory Table 217, it will look at gradually increasing or gradually decreasing values of A-X. To perform the rate smoothing function, a limit is placed on the number of increments that the control pointer may move at any one time. This action limits the amount or the rate at which the VAD and AVD values may change. Accordingly, at decision block 251 in Fig. 12A, a determination is made as to whether the pointer control has moved a maximum amount. If not, then the pointer may be moved one more address in order to look at the next value of A-X that is stored within the Memory Table 217. If the maximum movement of the pointer control has occurred, however, then further scanning of the memory table is terminated, and the A-X value stored at the then current pointer address is used as the updated $A-X_{Ref}$ value to be held in the A-X reference register, and the corresponding value of VAD and AVD are transferred to the pulse generator logic. In this fashion, the maximum rate at which the pacing interval (as set by the VAD and AVD values) may change can be controlled. Advantageously, this rate can be controlled whether it is increasing or decreasing. Moreover, through prescribing a different limit as the maximum amount which the pointer control may move as a function of whether the rate is increasing or decreasing, a hysteresis effect may be achieved.

Referring next to Figs. 13a and 13b, there are shown some illustrative graphs indicating some illustrative relationships between the API/ARI measurements and the corresponding AVD + VAD control parameters that may be programmed into the Memory Table 217. In Fig. 13a, it is seen that there may be a nominal AVD + VAD value corresponding to a measured and processed API value, as indicated by the line 260. This value may, of course, be programmed to move up or down vertically as a function of the particular patient, as indicated by the arrow 262. The left and right ends 264 and 266, respectively, of the line 260 may be programmed to bend up or down, as indicated by the arrows 268 and 270. In this fashion, any particular relationship desired may be programmed to exist between API and AVD + VAD. In Fig. 13b, Some possible relationships between the AR interval and the corresponding AVD + VAD pacing interval are also illustrated. A typical relationship may be as shown by line 275. However, because both the AVD and VAD values are stored in the Memory Table 217, it would be possible to program one to have an inverse relationship with respect to the measured ARI interval and the other to have a direct relationship, resulting in a pacing interval relationship as indicated by the dashed single-dot, line 277. As indicated previously, preliminary experiments indicate that the API measurement is increasing (getting longer) with increased workload on the patient. That is, as the heart rate needs to increase, the measured A-P interval is increasing. Because of the preliminary nature of these experiments, and the small number of samples that have been employed, further studies are being conducted. It is to be emphasized that the present invention is not limited to a particular relationship (increasing or decreasing) between the measured interval and the physiological needs of the patient. Rather, the present invention recognizes that there is a change in the measured API or ARI as this physiological need changes.

While the preferred embodiment of the physiological sensor of the present invention processes the measured A-X intervals in the manner described above, it is to be understood that other processing techniques or methods could be employed. For example, a simple moving average of the A-X intervals over a prescribed number of previous most recent consecutive cardiac cycles could be used in order to generate a smoothed reference A-X value. Alternatively, concurrent measurements of the AP and AR intervals could be compared to measurements of the PR interval (the time interval between a P-wave and an R-wave), and these various interval measurements could be compared one with another. Appropriate ratios, or relative changes between these measurements, could then be used to indicate a change in physiological need.

At least one embodiment of the invention includes compensation means for taking into account any artificially induced change in the A-X interval as a result of a change in pacing rate. That is, increasing or decreasing the rate at which an A-pulse is applied to the atrium, or a V-pulse is applied to the ventricle, may likely have some effect on the resultant A-P, A-R, or V-R intervals, regardless of any change in physiological need. This pacing-induced change in these time intervals can be measured for a given patient, and then compensation made therefor as a particular physiological profile of the patient is programmed into the Memory Table 217. For example, suppose a patient having an implanted pacemaker has a measured AP interval of 5Ø msec while the patient is at rest, and that the patient's heart rate is 7Ø ppm (pulses per minute). The pacemaker rate can be programmably increased by an attending physician until the patient's heart rate has increased to 1ØØ ppm, while the patient is still at rest. At a "resting" 1ØØ ppm, the AP interval could again be measured. Suppose it has increased to 6Ø msec. Next, the patient exercises an appropriate amount and the AP interval is again measured, and at an "exercised" 1ØØ ppm, the AP interval is measured to be, for example, 7Ø msec. The net difference between the "resting" 1ØØ ppm AP interval and the "exercise" 1ØØ ppm AP interval (i.e., 1Ø msec) would be the interval amount change that is properly attributable to physiological need, not the gross change in AP interval (i.e., 2Ø msec.) from the 7Ø ppm "resting" rate to the 1ØØ ppm "exercise" rate. It is this net difference, personalized for each patient, that is, in accordance with one embodiment of the invention, acted upon by the processing circuitry in order to detrmine true physiological need. Typically, compensation for the pacer-induced changes in the interval periods can occur by subtracting or adding a pre-determined number to the measured interval as a function of the pacer-interval, (i.e., heart rate), which pre-determined number can be calculated for each patient by the physician through some simple tests and measurements as described above.

Fig. 12B illustrates how the above described compensation technique could be included in the flow diagram of Fig. 12A. In Fig. 12B, the connecting A1 circle is intended to connect with the connecting circle 256 at the bottom of Fig. 12A, and the connecting A2 circle in Fig. 12B is intended to connect with the connecting circle 257 near the top of Fig. 12 A. The extended process shown in Fig. 12B includes a process step 258 for tracking any changes in the measured heart rate (measured by sensing the occurrence of P and R events through the pacer electrodes); and a process step 259 for compensating the stored AVD and VAD values by an appropriate amount (which could be stored in a separate section of the Memory Table). Alternatively, compensation of AVD and VAD could be performed externally prior to storing the AVD and VAD values in the Memory Table. Such a preprogrammed compensation is preferred if sufficient data can first be obtained from the patient.

Reference is now made to Figures 14A, 14B and 14C, which figures are state diagrams associated with operation of the preferred embodiment of the pulse generator logic 42 (Fig. 1Ø). What is shown in Figs. 14A-14C depicts a pacemaker that can operate in either the DDI or DDD modes. As mentioned previously, it is to be understood that the present invention is not limited to a pacemaker that can operate only in DDI or DDD modes. The state diagrams shown in Figs. 14A-14C are helpful in understanding the various timing sequences that are associated with the pulse generator logic. With the aid of such diagrams, those skilled in the art could readily realize the logic circuitry necessary to build the appropriate control logic.

With reference to the discussion that follows, the terminology of Table 1 should be noted.

TABLE 1

| Term | Definition of Term |
|---|---|
| TO | Time out of state preceeding reference |
| IRW | Inhibiting R wave |
| PW | P-wave |
| PNRE | P-wave noise refractory extension |
| IPW | Inhibiting P wave |
| RW | R-wave |
| RNRE | R-wave noise refractory extension |
| SAVE P | "Saved P" Pulse sensed during relative refractory period |
| API | A-P interval measurement |
| ARI | A-R interval measurement |
| VAD | V-A delay -- programmable |
| A | A-pulse |
| V | V-Pulse |
| ABS REF | Absolute refractory -- extendable if P-wave sensed |
| MTR | Maximum tracking rate interval - programmed |
| IPW | PW PNRE |
| PW | P DVI AVD ABS REF BLOCK BLANK A V |
| IRW | RE RNRE |
| RW | R ABS REF BLOCK BLANK A V |

Referring now to Fig. 14a, the state diagram associated with operation of the pulse generator logic 42 (Figs. 1∅) is indicated. Highlighted in Figure 14a, (in bold lines) is the path that the pulse generator logic would follow assuming that both an A-pulse and a V-pulse are generated. This will now be explained. Beginning with the VAD state 3∅∅, which state indicates that the V-A delay (a programmable delay) is in the process of timing out, the sequence is initiated. When VAD has timed out, and in the absence of IPW and SAVE P, an A pulse is generated as indicated at 3∅2. After a blanking period, at 3∅4, of approximately 11 msec, followed by a blocking period, at 3∅6, of approximately 2 msec, an AV delay, or AVD, is initiated at 3∅8. The A-V delay is programmable between approximately 125 to 175 msec. At the conclusion of this delay and in the absence of IRW, a V-pulse is generated at 31∅. When this is completed, there is a further blanking period, at 112, of 11 msec, followed by a blocking period, at 314 of 2 msec, before an Absolute Refractory Period, at 316, is initiated. When the Absolute Refractory Period has timed out, the A Relative Refractory Period begins, as indicated at 318. Once this period has timed out, an interval that in combination with the preceding intervals defines a Maximum Tracking Rate (which interval defines a minimum tracking interval) is initiated, as indicated at 32∅. Once this interval has timed out, and in the absence of either SAVE P or DDD, the V-A delay period is again initiated back at 3∅∅. Thus, a timing cycle or pacing interval is defined as the pulse generator logic moves through these various states. A timing line indicating these various pacing interval events is also included in Fig 14A. It is noted that the pacing interval comprises the VA interval and the AV interval, each interval of which may contain fixed time intervals and variable time intervals. The fixed timed intervals are programmable through appropriate telemetry controls, or (as is the case with the present invention) through receipt of new VAD or AVD values from the physiological detector.

Fig. 14B is similar to 14A except that no A-pulse is generated because IPW (inhibit P-wave) is present, indicating that a sinus P-wave has been sensed. Fig. 14B assumes that the DDI mode has been enabled, meaning that even though a sinus P-wave has been sensed, as indicated by the presence of SAVE P, the V-A delay is allowed to time out before the A-V delay begins. Once the A-V delay begins, the states change in the same manner as described in connection with Fig. 14A.

Fig. 14C is also similar to Figs. 14A and 14B, except that only an A-pulse is generated, not a V-pulse. In this figure, during the A-V delay, at 3∅8, IRW is generated, indicating that a naturally occuring R-wave has been sensed. Hence, there is no need to generate a V-pulse in order to stimulate the ventricle.

It should be evident from Figs. 14A-14C that only a small number of the various possible types of timing cycles have been illustrated. However, as those skilled in the art will recognize, the state diagrams of these figures include therein a large number of such possible combinations. It should also be emphasized that the state diagrams of Figs. 14A-14C are only representative of the types of state diagrams that may be employed with programmable pacemakers. It is again emphasized that the present invention is not directly

16

concerned with the type of state diagram that is employed, but rather with a means for adjusting VAD and AVD with the goal of adjusting the pacing interval so that the heart rate can be adjusted as a function of sensed physiological need. As such, it will be apparent that other timing intervals (besides VAD and AVD) included within the cycles shown in Figs. 14A-14C could be adjusted as a function of the API, ARI, or VRI measurements in order to adjust the pacing interval, and hence, the heart rate.

Figure 15 depicts some further state diagrams that relate to the state diagrams of Figures 14A-14C. Terminology used in connection with the diagrams of Fig. 15 is also found in Table 1. Fig. 15A, for example, illustrates that the SAVE P state is entered by the generation of an IPW signal (which signal is generated whenever a P-wave is sensed). Similarly, this state is disabled at the end of the absolute refractory period, or ABS REF.

Figs. 15B and 15C illustrate the R-wave noise refractory extension state, RNRE, and the P-wave noise refractory extension state, PNRE. The enabling of these states determines whether the IPW and IRW signals are generated, as indicated in Table 1.

Referring to Figures 15D and 15E, two additional states are shown that are used in conjunction with one embodiment the present invention. The API state is a state wherein the A-P interval measurement is enabled. This state is illustrated in Fig. 15D. Similarly, the ARI state is a state that indicates that the A-R interval measurement is enabled. This is illustrated in Fig: 15E. From Fig. 15D, it is seen that the API state, once enabled, is disabled if a time out occurs or if an IPW signal is generated, which IPW signal indicates that a sinus P-wave has been sensed. A time out period is assigned to the API state in order to account for the possibility that a paced P-wave may not occur in response to an A-pulse, (i.e., P-wave capture does not occur) or in case there is a failure to sense the P-wave for whatever reason. Further, in some patients, there may be several heart cycles where no P-wave occurs.

Appendix B, submitted herewith, provides additional information relative to incorporating the teachings of the present invention into a multi-mode programmable pacemaker.

Given the above description of the present invention, one skilled in the art could readily design and realize the appropriate circuitry for carrying out the invention. It is emphasixed that the actual circuit details associated with the pacemaker design are not critical to an understanding or use of the present invention. Rather, any suitable scheme or circuitry that allows A-P, A-R, or V-R measurements to be made, and then processes these measurements in order to adjust the various timing intervals that control the pacing rate, could be employed, and would fall within the scope of the appended claims. As indicated previously, additional background and details associated with the invention may be found in Appendices A and B, filed as a part hereof.

## APPENDIX A

### MICROPROCESSOR-CONTROLLED EMBODIMENT

Described herein is an alternative embodiment of the invention disclosed in the attached specification that is realized using a microprocessor and related circuitry.

With reference to the block diagram of Figure 10 of the specification, the embodiment described in this Appendix A includes relevant circuitry of the pulse generator logic 42, the interval measurement circuitry 71, and the physiological detector 73.

Figure A-1 depicts the overall block diagram of this alternative embodiment. A 1 msec clock source 402 delivers a 1 msec clock signal to a state timer 404. The state timer 404 receives state signals from a state signal generator 406, and control signals from a microprocessor 408. The microprocessor 408 is controlled by a program stored in ROM 410. A 1 msec counter 412, connected to the 1 msec clock source 402, is used to present appropriate timing signals to the microprocessor 408. The state timer 404 generates a strobe signal that is presented to a demultiplexer circuit 414. This strobe signal controls when various timing signals are presented to the state signal generator 406. The state signal generator 406, in response to the timing signals received, generates a set of state signals that define the particular operating state of the pacemaker. Possible operating states are explained in the specification in connection with Figures 14-15. Pulse amplifiers 416, in response to signals received from the demultiplexer 414, generate the appropriate stimulation pulses that are delivered to the heart. Sense ampliers 418 sense cardiac events that occur in the heart and present this information to the microprocessor 408.

Figure A-2 depicts a logic diagram of the state timer 404. Any appropriate logic circuitry could be employed, although CMOS is preferred because of its low power consumption. The device numbers shown in Figure A-2 (and in the other figures) represent commercially available components that can be purchased from numerous vendors, such as RCA, Motorola, and others.

Figure A-3 shows the preferred realization of the demultiplexer 414. As can be seen from this figure, two commercially available demultiplexer circuits are used, both of which are steered by the state signals generated by the state signal generator 406. However, only one of the demultiplexer curcuits is strobed with the strobe signal generated by the state timer 404. As those skilled in the art recognize, a demultiplexer circuit selects one of a plurality of possible output signals as selected by the data input (state) signals, and in synchrony with any strobe signal that may be present. In the absence of a strobe signal (and assuming the strobe signal has assumed a proper logic level), the selected output is enabled for so long as the data input signals selecting that particular output remain unchanged.

Figures A-4 and A-5 illustrate the logic diagram of the state signal generator 406. As shown in these figures, this circuit is realized using commmercially available logic gates and flip-flops. The signal names used in connection with Figures A-1 through A-4 are defined in Table 1 (in the specification) or are natural extensions of the definitions theregiven.

Table A-1 , shown below, lists the various states associated with the state timer 404 (Figure A-1) and the operating parameters associated with each.

The microprocessor 408 (Figure A-1) may be realized using any suitable microprocessor. In the preferred embodiment, an 8-bit commercially available MC146805 low power CMOS microprocessor, manufactured by Motorola, Inc., is used. No special techniques are involved in using the microprocsessor beyond what would be known to those skilled in the art. The program that controls the microprocessor is illustrated in the flow diagram of Figure A-6. A representative program listing, with comments, is listed in Table A-2. It is noted that for simplicity of understanding the program languages used in Table A-2 is a modified form of the "basic" programming language that could be readily converted to the requisite assembly language and its corresponding hex object code needed to control the MC146805 by those skilled in the art.

# TABLE A-1

| STATE | NAME | TIME/RANGE | INCREMENT | CLOCK | DIVIDED BY: |
|---|---|---|---|---|---|
| Ø | VAD | 0-1392/0 | 25 MS | 25 MS | 1-36 |
| 2 | ABLANK | 11 MS / 1 | — | 1 MS | 11 |
| 3 | ABLOCK | 2 MS / 2 | — | 1 MS | 2 |
| 4 | AVD | 125-175/ 1 | 25 MS | 25 MS | 5-7 |
| 6 | VBLANK | 11 MS/ 1 | — | 1 MS | 1 |
| 7 | VBLOCK | 2 MS / 2 | — | 1 MS | 2 |
| 8 | ABS REF | 100 MS / 4 | — | 25 MS | 4 |
| 9 | AREL REF | 100 MS / 4 | — | 25 MS | 4 |
| A | MTR | 200-600/5 | 25 MS | 25 MS | 8-24 |

20

TABLE A-2

| PROGRAM STATEMENT/FUNCTION | REMARKS |
|---|---|
| **MAIN PROGRAM** | |
| R1 = AX REF | SET BASELINE RATE |
| R2 = AV REF | |
| R3 = VA REF | |
| C1 = 0 | INITIALIZE CONSTANTS |
| C4 = 0 | |
| A2 = 0 | |
| A3 = 0 | |
| S2 = 1 | |
| S3 = -1 | |
| P = 1 | |
| TIME = 0:CLOCK 1 | ENABLE INTERRUPT |
| CALL RESET/START COUNTER SUB. | ENABLE INTERVAL COUNTER |
| WAIT | WAIT FOR INTERRUPT |
| | CONDITION (SPONTANEOUS |
| | P-WAVE, SENSED R-WAVE, OR |
| | GENERATED A OR V PULSE) |
| END | |
| **INTERRUPT PROGRAM** | |
| READ BITS (8 or 9) | DETERMINE IF V-P OR |
| | R-P INTRLV, THEREFORE NO A |
| | WAVE |
| IF (B9 OR B10.NE.1) GOTO PN10 | IF BB OR B9 SET, DO |
| | NOT GOTO AX INTRV PROGRAM |
| READ R-P OR V-P INTRVL INTO Al | INTRVL VALUE, BITS 2-9 OF |
| | COUNTER, LOADED INTO Al |
| CALL RESET/START COUNTER SUB. | RESET COUNTER SUBROUTINE |
| D4 = R3 - Al | SETTING CONSTANTS TO |
| | DETERMINE RATE AND |
| | DIRECTION OF CHANGE |
| C3 = SGN(D4) | |
| IF (C3.GT.0) THEN C2 = 100 | SETTING CONSTANTS FOR TABLE |
| ELSE C2 = 0 | LOOKUP |
| Al = 0 | |
| GOTO PN20 | BYPASS AX INTRVL PROGRAM |

| PROGRAM STATEMENT/FUNCTION | REMARKS |
|---|---|
| PN10 CONTINUE | AX INTRVL PROGRAM |
| READ (AX INTRVL) INTO A1 | LOAD BITS B0-B7 INTO REG A1 |
| CALL RESET/START COUNTER SUB. | |
| IF (C4.EQ.0) GOTO PN50 | CHECKING FOR SIGNIFICANT CHANGE |
| D1 = R1 - A1 | |
| SI = SGN D1 | CHECKS FOR DIRECTION OF CHANGE FROM REF. INTRVL AND LAST INTRVL MEASURED |
| TI=S2 * S1 | |
| IF (TI.EQ.-1) GOTO PN50 | CHECKS TO SEE IF CHANGES FROM 1ST INTRV AND 2ND INTRV ARE IN SAME DIRECTION |
| IF (T2. EQ-1) GOTO PN50 | |
| T3 = S3*S1 | |
| IF (T3.EQ-1) GOTO PN50 | |
| IF (ABS(D1).LT.ABS(C1)) THEN | SETTING MAX RATE CHANGE ALLOWED |
| D1 = C1 | |
| C2 = R1 - D1 | MAX CHANGE FOR TABLE LOOKUP |
| C3 = S1 | |
| PN20 IF (C3.GT.0) GOTO PN30 | CHECK FOR INCREASE/DECREASE IN RATE |
| PN21 IF (P.EQ.MAX) GOTO PN40 | OUT OF TABLE, NO MORE VALUES |
| IF (X(P+1).GT.C2) GOTO PN40 | |
| IF ((ABS(V(P+1))-R3).GT.VAMAX) GOTO PN40 | IF RATE CHANGE TOO MUCH, STOP AT PRESENT LOCATION ON TABLE |
| P = P+1 | MOVE POINTER UP TO NEW VALUE |
| GOTO PN21 | CONTINUE WORKING UP TABLE |
| PN30 IF (P.EQ.MIN) GOTO PN40 | GONE TOO FAR, EXIT TABLE LOOKUP |
| IF ((X(P-1).LT.C2) GOTO PN50 | CHECK TO SEE IF OUT OF RANGE ON TABLE |
| IF (ABS(V(P-1)-R3).GT.VAMAX) GOTO PN40 | MAX RATE CHANGE, EXIT TABLE LOOKUP |
| P = P-1 | MOVE POINTER DOWN TO NEW VALUE |
| GOTO PN30 | CONTINUE WORKING DOWN TABLE |
| PN40 R1 = X(P) | SET AX INTRV IN REF |
| R2 = I(P) | SET AV INTRV |
| R3 = V(P) | SET VA INTRV |

PROGRAM STATEMENT/FUNCTION

        OUTPUT R1, R2, R3

PN50 CONTINUE
     A3 = A2


     S2 = S1
     D3 = R1 - A3
     T2 = S2*SGN(D3)
     IF (ABS(D1).GT.ABS(D3))
         THEN C1 = D3
         ELSE C1 = D1
     A2 = A1
     C4 = A2 * A3
     RETURN

PROGRAM KEY

R1 - REFERENCE AX INTRV
R2 - REVERENCE AV INTRV
R3 - REFERENCE VA INTRV
A1 - 1ST MEASURED INTRV AX, OR R-P
     OR V-P
D1 - DIFFERENCE BETWEEN LAST A1 AND
     AX REF


S1 - SIGN OF D1
A2 - 2ND MEASURED INTRV AX

D2 - DIFFERENCE BETWEEN A2 AND AX
     REF
S2 - SIGN OF D2


A3 - 3RD MEASURED INTRV AX
D3 - DIFFERENCE BETWEEN A3 AND
     AX REF
T1, T2, T3 - HOLDS CHANGES OF
     (S1 TO S2), (S2 TO S3)
     AND (S1 TO S3)

REMARKS

SEND NEW VALUES TO STATE HRDWRE
SET UP FOR NEXT INTERRUPT
MOVE CALCULATED VALUES OF
LAST AX VALUE TO NEW
LOCATION


LOCATE SMALLEST CHANGE IN
AX OF LAST TWO CYCLES
(BEATS)

PROGRAM KEY (cont.)

P- TABLE POINTER
X(P) - AX VALUE IN TABLE
I(P) - AV VALUE IN TABLE
V(P) - VA VALUE IN TABLE

C4 - CONSTANT THAT HOLDS
SMALLEST PREVIOUS CHANGE IN
RATE OF CHANGES IN AX
VALUES

C3 - CONSTANT THAT HOLDS
TEMPORARY VALUES


C2 - CONSTANT THAT HOLDS
DIFFERENCE BETWEEN REF AX
VALUE AND MOST RECENT AX
VALUE MEASURED

C1 - CONSTANT THAT HOLDS
TEMPORARY VALUES

## APPENDIX B

## INTEGRATION OF INVENTION WITH A MULTI-MODE
## DDD, DDI, DVI, and VVI PACEMAKER

The following description provides additional detail relative to how the present invention is integrated into a four mode pacemaker offering DDD, DDI, DVI, and VVI modes of operation. In the preferred configuration, pacing in the atrium is unipolar (tip with respect to case) or bipolar; pacing and sensing in the ventricle is bipolar; and pacing and sensing in the atrium is bipolar or unipolar depending upon the sensing configuration used. That is, when in the normal DDD, DDI, DVI, or VVI modes of operation (API and ARI not enabled), both sensing and pacing is bipolar. However, in any of the dual chamber modes with API enabled, sinus P-wave sensing is bipolar, atrial pacing is unipolar tip to case, A-P interval sensing is unipolar ring to case, and the ventricular channel is fully bipolar. In the DDD or DDI mode, with the A-R interval measurement enabled, pacing and sensing in both chambers is bipolar. In VVI mode, pacing is unipolar tip; sensing is unipolar ring.

### 1. DDD MODE

### ATRIAL EVENT

The DDD mode of operation will first be explained. If a P-wave is sensed during the P-alert time (any non-refractory period), an AV delay (programmable from, for example, 125 to 175 msec) is initiated. If a P-wave is not sensed during the P-alert time, and the VA interval has expired, indicating that it is time for an A-pulse, an A-pulse is generated, followed by an 11 msec blanking period wherein the V and A sense amplifier inputs are isolated from the leads, followed by a 2 msec blocking period wherein all P and R sensed events are ignored. Following these blanking and blocking periods, an AV delay, less 13 msec (to account for the blanking and

blocking periods), is initiated.

## A-V DELAY

If an A-P interval measurement is enabled during the AV delay, the venticular sensed channel is alert and the atrial channel is refractory in the sense of usual DDD timing. That is, if a P-wave initiated an AV delay, then the atrial channel is refractory. If the AV delay was due to an A-pulse having been generated then, after the blocking period, an A-P interval measurement period of, for example, 50 to 100 msec (during the AV delay), is initiated. If, during this period, a P-wave is detected (ring to case measurement) then the period measured is stored and processed as suggested below or as previously described in connection with Figs. 11 and 12 (see specification) or as described in Appendix A. If the A-P interval measurement is not enabled, then the atrial channel is refractory and the ventricle channel is alert.

## A-P INTERVAL TO RATE AVERAGING ALGORITHM

An examplary alogrithm for controlling rate based on A-P interval will now be described. To control rate as a function of A-P interval this function must be enabled, the A-pulse must not have been inhibited for the previous four pacing cycles and three in four consecutive A-P interval measurements must have been made. If an inhibiting P-wave occurs or more than one post A-pulse in four is missed, the pacing rate reverts to the programmed value. Rate is controlled as a function of the last three or four A-P Interval measurements made during the last four pacing cycles. To select a pacing rate the A-P intervals measured are averaged and applied to a rate control table. From this table, which may be as simple as a clamped linear function of A-P interval, a pacing rate is determined. Alternatively, the processes described in connection with Figures 11 and 12 in the specification, or Appendix A, may be used.

## A-R INTERVAL TO RATE AVERAGING

An example algorithm for controlling rate based on A-R interval will now be described. To control rate as a function of the A-R interval this function must be enabled, the A-pulse must not have been inhibited for the previous four pacing cycles and three in four consecutive A-R interval measurements must have been made. If an inhibiting P-wave occurs or more than one R-wave is missed, the pacing rate reverts to the programmed value. Rate is controlled as a function of the last three or four A-R Interval measurements made during the last four pacing cycles. To select a pacing rate the A-R intervals measured are averaged and applied to a rate control table. From this table, which may be as simple as a clamped linear function of A-R interval, a pacing rate is determined. Alternatively, the processes described in connection with Figures 11 and 12 in the specification, or Appendix A, may be used.

## V-R INTERVAL TO RATE AVERAGING ALGORITHM

This algorithm is similar to the A-P interval except A becomes V and P becomes R in all pulse or wave references.

## VENTRICULAR EVENT

When a spontaneous R-wave is sensed, the V-pulse is inhibited and an A and V absolute refractory period begins. As an example, this period may be 100 msec. If no R-wave is sensed during the AV delay, a V-pulse is initiated, and an A and V absolute refractory period begins. As an example these refractory periods may also be 100 msec.

## V-A INTERVAL

During the A and V absolute refractory period, P and R waves are ignored. When the A and V absolute refractory period has expired, a relative refractory period begins. Any sensed P or R wave during the relative refractory period will initiate an extension of that period. If during this

extended period, another P or R wave is detected, a further extension of the period occurs. This excludes tracking on noise bursts or continuous noise. That is, if a relative refractory period is extended to the end of the V-A interval, then an A-pulse is generated as if no P or R wave was detected.

After the refractory period, a maximum tracking rate interval (MTR) is initiated during which the P and R wave sense logic is enabled, but during which sensed P or R waves are stored and not acted upon until the MTR has timed out. After the MTR, the P and R wave sense logic is also enabled, and if a P-wave is detected, the timers are set to the previously described state following an A-pulse or an inhibiting P-wave. If an R-wave is detected, the timers are set to the previously described state following a V-pulse or an inhibiting R-wave. If there are stored P or R waves detected during MTR, they are acted on as if they occurred just after MTR with the last stored event taking precedence in case both occurred.

2. DDI MODE

The DDI mode of operation is similar to the above described DDD mode except that if a P-wave is sensed during the P-wave alert period, the timers are not set as described above, but continue as if the P-wave did not occur. The only effect of the sensed P-wave is to cause the next scheduled A-pulse to not be generated.

3. DVI MODE

The DVI mode is similar to the DDI mode except that P-waves are blocked at all times and only A-R or V-R measurement is available.

4. VVI MODE

The VVI mode is similar to the DVI mode except that no atrial pacing can occur and only V-R measurement is available.

## Claims

**1.** A rate adaptive pacemaker (16) having means (42, 44;22, 24;30, 36) for generating and delivering stimulation pulses on demand and at a programmable rate, physiological response means (71, 73) for adjusting said rate as a function of an interval between the generation of a stimulation pulse and the occurrence of a responsive cardiac event, **characterized in** that said physiological responsive means (71, 73) comprises:

sensing means (48, 54, 56) for sensing the occurrence of a cardiac depolarization event, referred to as X, which depolarization event follows the delivery of a stimulation pulse, referred to as S, to a heart (18);

timing means (71) for measuring the S-X time interval between the generation of said stimulation pulse S and the occurrence of said sensed cardiac depolarization event X;

means (73) for automatically adjusting the rate at which the stimulation pulses are delivered to the heart as a function of said S-X time interval measured by said timing means (71).

**2.** A pacemaker according to claim 1, **characterized in** that said rate adjusting means (73) comprises: processing means (201 - 219) for deriving a reference time interval value from the S - X time intervals measured during a prescribed number of, at least three, consecutive cardiac cycles, whereby said rate adjusting means (73) adjusts the rate at which the stimulation pulses are delivered to the heart as a function of said reference time interval value.

**3.** A pacemaker according to claim 2, **characterized in** that said processing means (201 - 219) comprises means for averaging the S - X time intervals to derive said reference time interval value.

**4.** A pacemaker according to claim 2, **characterized in** that said processing means (201 - 219) includes trend identification means (205 - 217) for determining whether all said S - X time intervals are greater than or less than the reference time interval value representative of the current stimulation pulse delivery rate, and replacement means (205, 209, 211, 215, 217) for replacing, if said trend is present, the reference time interval value by a new reference time interval value corresponding to the S - X time interval closest to the reference time interval value, whereby the replacement speed is limited by smoothing means (215).

**5.** A pacemaker according to claim 2, 3 or 4, **characterize in** that said processing means includes compensation means (217) to compensate for a change in said S - X time interval due to a change in rate for said stimulation pulses.

**6.** A pacemaker according to claim 1, 2, 3, 4 or 5, **characterized in** that S is an atrial stimulation pulse, referred to as A, and X is an atrial depolarization event or a ventricular depolarization event referred to as P and R, respectively, where P denotes a P-wave and R denotes an R-wave, and said measured time interval S- X is the time interval between A and P or the time interval between A and R.

**7.** A pacemaker according to claim 1, 2, 3, 4 or 5, **characterized in** that S is a ventricular stimulation pulse, referred to as V, and X is a ventricular depolarization event referred to as R, where R denotes an R-wave, and said measured time interval S - X is the time interval between V and R.

## Patentansprüche

**1.** Frequenzgesteuerter Herzschrittmacher (16) mit Mitteln (42,44;22,24;30,36) zur Erzeugung und Abgabe von Stimulationsimpulsen bei Bedarf und mit programmierbarer Frequenz, physiologische Ansprechmittel (71,73) zur Einstellung der Frequenz als Funktion eines Intervalls zwischen der Erzeugung eines Stimulationsimpulses und dem Auftreten eines Antwortereignisses des Herzens, **dadurch gekennzeichnet**, daß die physiologischen Ansprechmittel (71,73) folgendes umfassen:

Abtastmittel (48,54,56), die das Auftreten einer als X bezeichneten Depolarisation des Herzens erfassen, wobei diese Depolarisation der Abgabe eines als S bezeichneten Stimulationsimpulses an das Herz folgt; zeitmessende Mittel (71), die das Zeitintervall S-X zwischen der Erzeugung des Stimulationsimpulses S und dem Auftreten der erfaßten Depolarisation X messen; Mittel (73) zur automatischen Einstellung der Frequenz der an das Herz abgegebenen Stimulationsimpulse als Funktion des durch die zeitmessenden Mittel (71) gemessenen Zeitintervalls S-X.

**2.** Herzschrittmacher nach Anspruch 1, **dadurch gekennzeichnet**, daß die Mittel (73) zum Einstellen der Frequenz folgendes umfassen:

Prozeßmittel (201-209) zur Ableitung eines Bezugszeitintervalls aus den Zeitintervallen S-X, die während einer vorbestimmten Anzahl, mindestens drei, aufeinanderfolgenden Herzzyklen gemessen werden, wobei die Mittel (73) zum Einstellen der Frequenz die Frequenz der an das Herz abzugebenden Impulse als Funktion des Bezugszeitintervalls einstellen.

**3.** Herzschrittmacher nach Anspruch 2, **dadurch gekennzeichnet**, daß die Prozeßmittel (201-219) Mittel zur Bildung des Mittelwertes der Zeitintervallen S-X enthalten, um das Bezugszeitintervall abzuleiten.

**4.** Herzschrittmacher nach Anspruch 2, **dadurch gekennzeichnet**, daß die Prozeßmittel (201-219) folgendes umfassen:

trendidentifizierende Mittel (205-217) zur Feststellung, ob alle Zeitintervalle S-X größer oder kleiner sind als das der geltenden Abgabefrequenz der Stimulationsimpulse entsprechende Bezugszeitintervall und Austauschmittel (205,209,211,215,217) zum Austauschen des Bezugszeitintervalls mit dem Zeitintervall S-X, das dem Bezugszeitintervall am nächsten kommt, wobei die Austauschgeschwindigkeit durch Glättungsmittel (215) begrenzt ist.

**5.** Herzschrittmacher nach Anspruch 2,3 oder 4, **dadurch gekennzeichnet**, daß die Prozeßmittel (201-219) Kompensationsmittel (217) zur Kompensierung einer Änderung im Zeitintervall S-X infolge einer Frequenzänderung für die Stimulationsimpulse enthalten.

**6.** Herzschrittmacher nach Anspruch 1,2,3,4 oder 5, **dadurch gekennzeichnet**, daß S ein als A bezeichneter atrieller Stimulationsimpuls und X eine als P oder beziehungsweise als R bezeichnete atrielle oder ventrikuläre Depolarisation ist, wobei P eine P-Welle und R eine R-Welle bezeichnet und wobei das gemessene Zeitintervall S-X das Zeitintervall zwischen A und P oder zwischen A und R ist.

**7.** Herzschrittmacher nach Anspruch 1,2,3,4 oder 5, **dadurch gekennzeichnet**, daß S ein als V bezeichneter ventrikulärer Stimulationsimpuls und X eine als R bezeichnete ventrikuläre Depolarisation ist, wobei R eine R-Welle bezeichnet und das gemessene Zeitintervall S-X das Zeitintervall zwischen V und R ist.

**Revendications**

**1.** Un stimulateur (16) à cadence adaptative comportant des moyens (42, 44; 22, 24; 30, 36) destinés à générer et à fournir des impulsions de stimulation à la demande et à une cadence programmable, et des moyens sensibles à une réponse physiologique (71, 73) qui sont destinés à régler la cadence précitée en fonction d'un intervalle entre la génération d'une impulsion de stimulation et l'apparition d'un événement cardiaque qui est une réaction à cette impulsion, **caractérisé** en ce que les moyens sensibles à une réponse physiologique (71, 73) comprennent :

des moyens de détection (48, 54, 56) destinés à détecter l'apparition d'un événement de dépolarisation cardiaque, que l'on appelle X, cet événement de dépolarisation faisant suite à l'application à un coeur (18) d'une impulsion de stimulation que l'on appelle S;

des moyens de mesure de temps (71) destinés à mesurer l'intervalle de temps S-X entre la génération de l'impulsion de stimulation S et l'apparition de l'événement de dépolarisation cardiaque X détecté; et des moyens (73) destinés à régler automatiquement la cadence à laquelle les impulsions de stimulation sont appliquées au coeur, en fonction de l'intervalle de temps S-X qui est mesuré par les moyens de mesure de temps (71).

**2.** Un stimulateur selon la revendication 1, **caractérisé** en ce que les moyens de réglage de cadence (73) comprennent :

des moyens de traitement (201-219) destinés à déterminer une valeur d'intervalle de temps de référence à partir des intervalles de temps S-X qui sont mesurés pendant un nombre déterminé, au moins égal à trois, de cycles cardiaques consécutifs, grâce à quoi les moyens de réglage de cadence (73) règlent la cadence à laquelle les impulsions de stimulation sont appliquées au coeur, en fonction de la valeur d'intervalle de temps de référence.

3. Un stimulateur selon la revendication 2, **caractérisé** en ce que les moyens de traitement (201-219) comprennent des moyens qui sont destinés à calculer la moyenne des intervalles de temps S-X pour déterminer la valeur d'intervalle de temps de référence.

4. Un stimulateur selon la revendication 2, **caractérisé** en ce que les moyens de traitement (201-219) comprennent des moyens d'identification de tendance (205-217) qui sont destinés à déterminer si tous les intervalles de temps S-X sont supérieurs ou inférieurs à la valeur d'intervalle de temps de référence qui est représentative de la cadence d'application d'impulsions de stimulation courante, et des moyens de remplacement (205, 209, 211, 215, 217) qui sont destinés à remplacer, si la tendance précitée existe, la valeur d'intervalle de temps de référence par une nouvelle valeur d'intervalle de temps de référence correspondant à l'intervalle de temps S-X le plus proche de la valeur d'intervalle de temps de référence, grâce à quoi la vitesse de remplacement est limitée par des moyens de lissage (215).

5. Un stimulateur selon la revendication 2, 3 ou 4, **caractérisé** en ce que les moyens de traitement comprennent des moyens de compensation (217) qui sont destinés à compenser un changement de l'intervalle de temps S-X qui est dû à un changement de cadence des impulsions de stimulation.

6. Un stimulateur selon la revendication 1, 2, 3, 4 ou 5, **caractérisé** en ce que S est une impulsion de stimulation auriculaire, que l'on désigne par A, et X est un événement de dépolarisation auriculaire ou un événement de dépolarisation ventriculaire, que l'on désigne respectivement par P et R, P désignant une onde P et R désignant une onde R, et l'intervalle de temps mesuré S-X est l'intervalle de temps entre A et P, ou l'intervalle de temps entre A et R.

7. Un stimulateur selon la revendication 1, 2, 3, 4 ou 5, **caractérisé** en ce que S est une impulsion de stimulation ventriculaire, que l'on désigne par V, et X est un événement de dépolarisation ventriculaire que l'on désigne par R, R désignant une onde R, et l'intervalle de temps mesuré S-X est l'intervalle de temps entre V et R.

# FIG. 1

# FIG. 2

# FIG. 3

ONE HEART CYCLE
OR "BEAT"

## FIG. 4

## FIG. 8

## FIG. 5

ONE HEART CYCLE

A    V    T    A    V

$P_P$

$R_P$

PACING INTERVAL

## FIG. 6

PACING INTERVAL

R    R

$A_1$    $P_P$    $A_2$    $P_P$

$API_1$    $API_2$

$ARI_1$    $ARI_2$

34

# FIG. 7A

# FIG. 7B

# FIG. 9A

# FIG. 9B

FIG. 10

EP 0 220 194 B1

FIG. 11

EP 0 220 194 B1

FIG. 12A

# FIG. 12B

MEASURED HEART · RATE → | MONITOR CHANGE IN HEART RATE | ⌐258

| COMPENSATE "AVD" AND "VAD" AS REQUIRED | ⌐259

(A₁)

(A₂)

FIG. 13A

API
(VRI)

AVD + VAD

FIG. 13B

ARI

AVD + VAD

# FIG. 14A

EP 0 220 194 B1

FIG. 14B

$TO \cdot (\overline{SAVEP} + \overline{DDD})$

$TO \cdot SAVEP \cdot DDD$

V ~310

DONE → BLANK ~312

DONE → BLOCK ~314

TO

$TO \cdot SAVEP \cdot DDI$

300 ~ VAD

$IPW \cdot DDD$

AVD ~308

IRW →

ABS REF ~316

TO →

A REL REF ~318

TO →

MTR ~320

DONE

$TO \cdot \overline{IPW} \cdot \overline{SAVEP}$

DONE

~302 A

DONE → BLANK ~304

DONE → BLOCK ~306

IRW

PACING INTERVAL

VAI

AVI

BLANK

BLOCK

ABS REF | A REL REF | MTR | VAD | AVD

EP 0 220 194 B1

FIG. 14C

EP 0 220 194 B1

# FIG. 15

EP 0 220 194 B1

ABSREF

SAVE P   SAVE P

IPW

FIG. 15A

TO

RNRE   $\overline{RNRE}$

RW·RNRE+END ABS REF

FIG. 15B

TO

PNRE   $\overline{PNRE}$

PW·(PNRE+A REL REF)

FIG. 15C

TO+IPW

API   $\overline{API}$

AVI ON·END OF BLOCK,
START OF AVD

FIG. 15D

V+IRW

ARI   $\overline{ARI}$

A·ARI ON

FIG. 15E

EP 0 220 194 B1

FIG. 16A

FIG. 16B

46

# FIG. 17A

## FIG. 17B

EP 0 220 194 B1

EP 0 220 194 B1

FIG. 18

FIG. 20

49

# FIG. 19

P-WAVE SENSE/PACE AMPLIFIER RESPONCE 113
(AMPLIFIER 48)

P-WAVE SENSING ECG AMPLIFIER RESPONSE 114 (APMLIFIER 54)

115'

3db

ATTENUATION

R-WAVE AND P-WAVE FREQUENCY

T-WAVE VENTRICAL FREQUENCY

MUSCLE FREQUENCY

115

116

3Hz    10Hz    60Hz    100Hz    125Hz

FREQUENCY (NOT TO SCALE) ——▶

EP 0 220 194 B1

# FIG. A-1

STATE
SIGNAL
GENERATOR

STATE
SIGNALS

DEMUX
STROBE

TIMING SIGNALS

406

414

A/V

404

PROGRAMMED
TIMER

PULSE
AMPS

STATE
STATUS

CONTROL

416

TO/FROM
HEART

408

MICROPROCESSOR
MC146805

PROGRAM
ROM

SENSE
AMPS

418

402

412

410

1 MSEC
CLOCK

COUNTERS

FIG. A-2

EP 0 220 194 B1

# FIG. A-3

# FIG. A-4

# FIG. A-5

FIG. A-6